# EUROPÄISCHE PATENTANMELDUNG

(11) **EP 2 272 846 A1**
(43) Veröffentlichungstag der Anmeldung: **12.01.2011**
(21) Anmeldenummer: 09163473.3
(22) Anmeldetag: 23.06.2009
(51) Int. Cl.: C07D 417/14, A01N 43/78

(54) **Thiazolylpiperidin Derivate als Fungizide**

(71) Anmelder: Bayer CropScience AG, 40789 Monheim (DE)
(72) Erfinder: Die Erfindernennung liegt noch nicht vor

(57) **Zusammenfassung**

Thiazolylpiperidin Derivate der Formel (**I**), 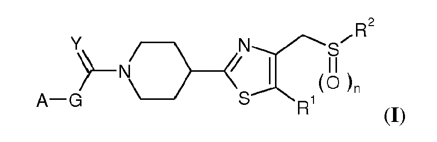 in welcher die Symbole A, G, Y, n, R¹, R², R³, R⁴, und R⁵ die in der Beschreibung angegebenen Bedeutungen haben, sowie agrochemisch wirksame Salze davon und deren Verwendung zur Bekämpfung von pflanzenpathogenen Schadpilzen sowie Verfahren zur **II**erstellung von Verbindungen der Formel (I).

## Beschreibung

Die Erfindung betrifft Thiazolylpiperidin Derivate, deren agrochemisch wirksamen Salze, deren Verwendung sowie Verfahren und Mittel zur Bekämpfung von pflanzenpathogenen Schadpilzen in und/oder auf Pflanzen oder in und/oder auf Saatgut von Pflanzen, Verfahren zur Herstellung solcher Mittel und behandeltes Saatgut sowie deren Verwendung zur Bekämpfung von pflanzenpathogenen Schadpilzen in der Land-, Garten- und Forstwirtschaft, in der Tiergesundheit, im Materialschutz sowie im Bereich Haushalt und Hygiene. Die vorliegende Erfindung betrifft weiterhin ein Verfahren zur Herstellung von Thiazolylpiperidin Derivaten.

Es ist bereits bekannt, dass bestimmte heterocyclisch substituierte Thiazole als fungizide Pflanzenschutzmittel benutzt werden können (siehe WO 07/014290, WO 08/013925, WO 08/013622, WO 08/091594, WO 08/091580, WO 09/055514). Die fungizide Wirksamkeit dieser Verbindungen ist jedoch gerade bei niedrigeren Aufwandmengen nicht immer ausreichend.

In WO 08/083238 werden bestimmte Thiazolylpiperidinsulfid- und sulfon-Derivate beschrieben, die ebenfalls medizinisch genutzt werden können, hier zur Behandlung von Diabetes und metabolischer Funktionsstörungen. Wirkung auf pilzliche Pathogene wird jedoch nicht beschrieben.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden Thiazolylpiperidin Derivate die genannten Aufgaben zumindest in Teilaspekten lösen und sich als Pflanzenschutzmittel, insbesondere als Fungizide eignen.

Gegenstand der Erfindung sind Verbindungen der Formel **(I),** 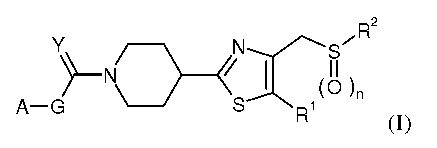 in welcher die Symbole folgende Bedeutungen haben:
- A: steht für Phenyl, das bis zu drei Substituenten enthalten kann,
wobei die Substituenten unabhängig voneinander ausgewählt sind aus folgender Liste: Cyano, Nitro, Halogen, C₁-C₆-Alkyl, C₁-C₄-Halogenalkyl, C₃-C₆-Cycloalkyl, C₃-C₆-Halogen- cycloalkyl, C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆-Halogenalkenyl, C₂-C₆-Alkinyl, C₂-C₆-Halogenalkinyl, Tri(C₁-C₄-al- kyl)silyl, Benzyl, Phenyl, Hydroxy, C₁-C₄-Alkoxy, C₁-C₄-Halogenalkoxy, OCH₂OCH₃, SH, C₁-C₄-Alkylthio, C₁-C₆-Halogenalkylthio, CHO, COOH, (C₁-C₄-Alkoxy)carbonyl, CONR³R⁴, CR³=NOR⁴, (C₁-C₄-Alkyl)carbonyl, (C₁-C₄-Halogenalkyl)carbonyl, (C₁-C₄- Alkyl)carbonyloxy, (C₁-C₄-Alkyl)carbonylthio, C₁-C₄-Alkylsulfinyl, C₁-C₄-Halogenalkyl- sulfinyl, C₁-C₄-Alkylsulfonyl, C₁-C₄-Halogenalkylsulfonyl, NR³R⁴, NR³COR⁴, SF₅, SO₂NR³R⁴, C₂-C₄-Alkoxyalkyl, oder 1-Methoxycyclopropyl, oder
- A: steht für einen heteroaromatischen Rest ausgewählt aus folgender Gruppe: Furan-2-yl, Furan- 3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Py- razol-4-yl, Imidazol-1-yl, Imidazol-2-yl, Imidazol-4-yl, Pyridin-2-yl, Pyridin-3-yl, Pyridin-4- yl, Pyridazin-3-yl, Pyridazin-4-yl, Pyrimidin-2-yl, Pyrimidin-4-yl oder Pyrimidin-5-yl, der bis zu drei Substituenten enthalten kann, wobei die Substituenten unabhängig voneinan- der ausgewählt sind aus folgender Liste:

### Substituenten am Kohlenstoff:

Cyano, Nitro, Halogen, C₁-C₆-Alkyl, C₁-C₄-Halogenalkyl, C₃-C₆-Cycloalkyl, C₃-C₆- Halogencycloalkyl C₄-C₁₀-Cycloalkylalkyl, C₄-C₁₀-Alkylcycloalkyl, C₅-C₁₀-Alkylcycloalkyl- alkyl, C₂-C₆-Alkenyl, C₂-C₆-Halogenalkenyl, C₂-C₆-Alkinyl, C₂-C₆-Halogenalkinyl, Tri(C₁- C₄-alkyl)silyl, Benzyl, Phenyl, Hydroxy, C₁-C₄-Alkoxy, C₁-C₄-Halogenalkoxy, OCH₂OCH₃, SH, C₁-C₄-Alkylthio, C₁-C₆-Halogenalkylthio, CHO, COOH, (C₁-C₄-Alkoxy)carbonyl, CONR³R⁴, CR³=NOR⁴, (C₁-C₄-Alkyl)carbonyl, (C₁-C₄-Halogenalkyl)carbonyl, (C₁-C₄- Alkyl)carbonyloxy, (C₁-C₄-Alkyl)carbonylthio, C₁-C₄-Alkylsulfinyl, C₁-C₄-Halogenalkyl- sulfinyl, C₁-C₄-Alkylsulfonyl, C₁-C₄-Halogenalkylsulfonyl, NR³R⁴, NR³COR⁴, SF₅, SO₂NR³R⁴, C₂-C₄-Alkoxyalkyl oder 1-Methoxycyclopropyl,

### Substituenten am Stickstoff:

Hydroxy, Cyano, NR³R⁴, C₁-C₆-Alkyl, C₁-C₄-Halogenalkyl, C₃-C₆-Cycloalkyl, C₃-C₆- Halogencycloalkyl, C₄-C₁₀-Alkylcyloalkyl, C₂-C₆-Alkenyl, C₂-C₆-Halogenalkenyl, C₂-C₆- Alkinyl oder C₂-C₆-Halogenalkinyl,
- G: steht für (C(R⁵)₂)ₚ, mit p = 1 oder 2,
oder
- G: steht für NH, unter der Voraussetzung, dass G an ein Kohlenstoffatom von A gebunden ist,
- Y: steht für Schwefel oder Sauerstoff,
- R¹: steht für Wasserstoff, C₁-C₂-Alkyl, C₁-C₂-Halogenalkyl oder Halogen,
- n =: 0 bis 2,
- R²: steht für C₁-C₈-Alkyl, C₁-C₄-Halogenalkyl, C₂-C₆-Alkenyl, C₂-C₆-Alkynyl, Adamantan-1-yl oder Adamantan-2-yl, oder
- R²: steht für unsubstituiertes oder substituiertes C₃-C₁₀-Cycloalkyl, wobei die Substituenten unabhängig voneinander ausgewählt sind aus folgender Liste: Cyano, Halogen, C₁-C₆-Alkyl, C₁-C₆-Halogenalkyl, C₃-C₆-Cycloalkyl, C₂-C₆-Alkenyl, C₂-C₆- Halogenalkenyl, C₂-C₆-Alkinyl, C₂-C₆-Halogenalkinyl, Tri(C₁-C₄-alkyl)silyl, Phenyl, Hydro- xy, Oxo, C₁-C₆-Alkoxy, C₁-C₆-Halogenalkoxy, C₂-C₆-Alkenyloxy, C₂-C₆-Alkinyloxy, C₁-C₆- Alkylthio oder C₁-C₆-Halogenalkylthio, oder
- R²: steht für unsubstituiertes oder substituiertes C₅-C₁₀-Cyloalkenyl, wobei die Substituenten unabhängig voneinander ausgewählt sind aus folgender Liste: Cyano, Halogen, C₁-C₆-Alkyl, C₁-C₆-Halogenalkyl, C₃-C₆-Cycloalkyl, C₂-C₆-Alkenyl, C₂-C₆- Halogenalkenyl, C₂-C₆-Alkinyl, C₂-C₆-Halogenalkinyl, Tri(C₁-C₄-alkyl)silyl, Phenyl, Hydro- xy, Oxo, C₁-C₆-Alkoxy, C₁-C₆-Halogenalkoxy, C₂-C₆-Alkenyloxy, C₂-C₆-Alkinyloxy, C₁-C₆- Alkylthio oder C₁-C₆-Halogenalkylthio,
oder
- R²: steht für unsubstituiertes oder substituiertes Phenyl, wobei die Substituenten unabhängig voneinander ausgewählt sind aus folgender Liste: Cyano, Nitro, Halogen, C₁-C₆-Alkyl, C₁-C₄-Halogenalkyl, C₃-C₆-Cycloalkyl, C₃-C₆-Halogen- cycloalkyl, C₄-C₁₀-Cycloalkylalkyl, C₄-C₁₀-Halogencycloalkylalkyl, C₄-C₁₀-Alkylcycloalkyl, C₅-C₁₀-Alkylcycloalkylalkyl, C₄-C₁₀-Cycloalkoxyalkyl, C₂-C₆-Alkenyl, C₂-C₆-Halogenalke- nyl, C₃-C₈-Cycloalkenyl, C₃-C₈-Halocycloalkenyl, C₂-C₆-Alkinyl, C₂-C₆-Halogenalkinyl, C₂- C₆-Alkoxyalkyl, C₂-C₆-Halogenalkoxyalkyl, C₃-C₈-Alkoxyalkoxyalkyl, Tri(C₁-C₄-alkyl)silyl, Benzyl, Phenyl, Hydroxy, C₁-C₆-Alkoxy, C₂-C₆-Alkoxyalkoxy, C₁-C₆-Halogenalkoxy, C₂- C₆-Alkenyloxy, C₂-C₆-Halogenalkenyloxy, C₂-C₆-Alkinyloxy, C₂-C₆-Halogenalkinyloxy, C₃- C₆-Cycloalkoxy, C₃-C₆-Cycloalkyloxy, C₃-C₆-Halocycloalkoxy, C₄-C₁₀-Cycloalkylalkyloxy, NR³R⁴, SH, SF₅, C₁-C₆-Alkylthio, C₁-C₆-Halogenalkylthio, C₃-C₆-Cycloalkylthio, CHO, COOH, (C₁-C₆-Alkoxy)carbonyl, CONR³R⁴, CR³=NOR⁴, (C₁-C₆-Alkyl)carbonyl, (C₁-C₆- Halogenalkyl)carbonyl, (C₁-C₆-Alkyl)carbonyloxy, (C₁-C₆-Halogenalkyl)carbonyloxy, (C₁- C₆-Alkyl)carbonylthio, C₁-C₆-Alkylsulfinyl, C₁-C₆-Halogenalkylsulfinyl, C₁-C₆-Alkyl- sulfonyl, C₁-C₆-Halogenalkylsulfonyl, NR³COR⁴ oder SO₂NR³R⁴, oder
- R²: steht für gesättigtes oder teilweise oder vollständig ungesättigtes, unsubstituiertes oder substi- tuiertes, Naphthyl oder Indenyl, wobei die Substituenten unabhängig voneinander ausgewählt sind aus folgender Liste: Cyano, Nitro, Halogen, C₁-C₆-Alkyl, C₁-C₄-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Benzyl, Phenyl, Hydroxy, C₁-C₆-Alkoxy, C₁-C₆-Halogenalkoxy, C₂-C₆-Alkenyloxy, C₂-C₆- Alkinyloxy, C₁-C₆-Alkylthio oder C₁-C₆-Halogenalkylthio, oder
- R²: steht für einen unsubstituierten oder substituierten 5- oder 6-gliedrigen Heteroarylrest, wobei die Substituenten unabhängig voneinander ausgewählt sind aus folgender Liste: Substituenten am Kohlenstoff: Cyano, Nitro, Halogen, C₁-C₆-Alkyl, C₁-C₃-Halogenalkyl, C₃- C₆-Cycloalkyl, C₃-C₆-Halogencycloalkyl, C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Benzyl, Phenyl, Hydroxy, C₁-C₄-ALkoxy, C₁-C₄- Halogenalkoxy, OCH₂OCH₃, SH, C₁-C₄-Alkylthio, C₁-C₆-Halogenalkylthio, COOH, (C₁-C₄- Alkoxy)carbonyl, CONR³R⁴, (C₁-C₄-Alkyl)carbonyl, (C₁-C₄-Halogenalkyl)carbonyl, (C₁-C₄- Alkyl)carbonyloxy, (C₁-C₄-Alkyl)carbonylthio, C₁-C₄-Alkylsulfinyl, C₁-C₄-Halogenalkyl- sulfinyl, C₁-C₄-Alkylsulfonyl, C₁-C₄-Halogenalkylsulfonyl, NR³R⁴, NR³COR⁴, SF₅, SO₂NR³R⁴, C₂-C₄-Alkoxyalkyl oder 1-Methoxycyclopropyl, Substituenten am Stickstoff: Cyano, C₁-C₆-Alkyl, C₁-C₃-Halogenalkyl, C₃-C₆-Cycloalkyl, C₃- C₆-Halogencycloalkyl, C₄-C₁₀-Alkylcyloalkyl, C₂-C₆-Alkenyl, C₂-C₆-Halogenalkenyl, C₂- C₆-Alkinyl, C₂-C₆-Halogenalkinyl oder Phenyl, oder
- R²: steht für benzokondensiertes unsubstituiertes oder substituiertes 5- oder 6- gliedriges Hetero- aryl, wobei die Substituenten unabhängig voneinander ausgewählt sind aus folgender Liste: Substituenten am Kohlenstoff: Cyano, Nitro, Halogen, C₁-C₆-Alkyl, C₁-C₃-Halogenalkyl, C₃- C₆-Cycloalkyl, C₃-C₆-Halogencycloalkyl C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆-Halogenalkenyl, C₂-C₆-Alkinyl, C₂-C₆- Halogenalkinyl, Tri(C₁-C₄-alkyl)silyl, Benzyl, Phenyl, Hydroxy, C₁-C₄-Alkoxy, C₁-C₄- Halogenalkoxy, OCH₂OCH₃, SH, C₁-C₄-Alkylthio, C₁-C₆-Halogenalkylthio, COOH, (C₁-C₄- Alkoxy)carbonyl, CONR³R⁴, (C₁-C₄-Alkyl)carbonyl, (C₁-C₄-Halogenalkyl)carbonyl, (C₁-C₄- Alkyl)carbonyloxy, (C₁-C₄-Alkyl)carbonylthio, C₁-C₄-Alkylsulfinyl, C₁-C₄-Halogenalkyl- sulfinyl, C₁-C₄-Alkylsulfonyl, C₁-C₄-Halogenalkylsulfonyl, NR³R⁴, NR³COR⁴, SF₅, SO₂NR³R⁴, C₂-C₄-Alkoxyalkyl oder 1-Methoxycyclopropyl, Substituenten am Stickstoff: Cyano, C₁-C₆-Alkyl, C₁-C₃-Halogenalkyl, C₃-C₆-Cycloalkyl, C₃- C₆-Halogencycloalkyl, C₄-C₁₀-Alkylcyloalkyl, C₂-C₆-Alkenyl, C₂-C₆-Halogenalkenyl, C₂- C₆-Alkinyl, C₂-C₆-Halogenalkinyl oder Phenyl, oder
- R²: steht für unsubstituiertes oder substituiertes C₅-C₁₅-Heterocyclyl, wobei mögliche Substituenten unabhängig voneinander ausgewählt sind aus folgender Liste: Substituenten am Kohlenstoff: Cyano, Nitro, Halogen, C₁-C₆-Alkyl, C₁-C₃-Halogenalkyl, C₃- C₆-Cycloalkyl, C₃-C₆-Halogencycloalkyl C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆-Halogenalkenyl, C₂-C₆-Alkinyl, C₂-C₆- Halogenalkinyl, Tri(C₁-C₄-alkyl)silyl, Benzyl, Phenyl, Hydroxy, C₁-C₄-Alkoxy, C₁-C₄- Halogenalkoxy, OCH₂OCH₃, SH, C₁-C₄-Alkylthio, C₁-C₆-Halogenalkylthio, COOH, (C₁-C₄- Alkoxy)carbonyl, CONR³R⁴, (C₁-C₄-Alkyl)carbonyl, (C₁-C₄-Halogenalkyl)carbonyl, (C₁-C₄- Alkyl)carbonyloxy, (C₁-C₄-Alkyl)carbonylthio, C₁-C₄-Alkylsulfinyl, C₁-C₄-Halogenalkylsul- finyl, C₁-C₄-Alkylsulfonyl, C₁-C₄-Halogenalkylsulfonyl, NR³R⁴, NR³COR⁴, SF₅, SO₂NR³R⁴, C₂-C₄-Alkoxyalkyl oder 1-Methoxycyclopropyl,
Substituenten am Stickstoff: Cyano, C₁-C₆-Alkyl, C₁-C₃-Halogenalkyl, C₃-C₆-Cycloalkyl, C₃- C₆-Halogencycloalkyl, C₄-C₁₀-Alkylcyloalkyl, C₂-C₆-Alkenyl, C₂-C₆-Halogenalkenyl, C₂- C₆-Alkinyl, C₂-C₆-Halogenalkinyl oder Phenyl,
- R³, R⁴: stehen unabhängig voneinander für Wasserstoff, C₁-C₄-Alkyl, C₁-C₃-Halogenalkyl, C₃-C₆- Cycloalkyl, Benzyl oder Phenyl,
- R⁵: ist gleich oder verschieden unabhängig voneinander Wasserstoff, C₁-C₂-Alkyl oder C₁-C₂- Halogenalkyl,
sowie agrochemisch wirksame Salze davon.

Ein weiterer Gegenstand ist die Verwendung der Verbindungen der Formel **(I)** als Fungizide.

Erfindungsgemäße Thiazolylpiperidin Derivate der Formel **(I)** sowie deren agrochemisch wirksame Salze eignen sich sehr gut als zur Bekämpfung pflanzenpathogener Schadpilze. Die vorgenannten erfindungsgemäßen Verbindungen zeigen vor allem eine starke fungizide Wirksamkeit und lassen sich sowohl im Pflanzenschutz, im Bereich Haushalt und Hygiene als auch im Materialschutz verwenden.

Die Verbindungen der Formel **(I)** können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Bevorzugt werden Verbindungen der Formel (I), in denen ein oder mehrere der Symbole eine der folgenden Bedeutungen haben:
- A: steht für Phenyl, das bis zu drei Substituenten enthalten kann, wobei die Substituenten unabhängig voneinander ausgewählt sind aus folgender Liste: Cyano, Nitro, Halogen, C₁-C₄-Alkyl, C₁-C₃-Halogenalkyl, C₃-C₆-Cycloalkyl, C₂-C₄-Alkenyl, C₂-C₄-Alkinyl, C₁-C₄-Alkoxy, C₁-C₃-Halogenalkoxy, C₁-C₄-Alkylthio, C₁-C₃-Halogenalkyl- thio, (C₁-C₄-Alkoxy)carbonyl, (C₁-C₄-Alkyl)carbonyl, (C₁-C₃-Halogenalkyl)carbonyl, C₁-C₄- Alkylsulfinyl, C₁-C₃-Halogenalkylsulfinyl, C₁-C₄-Alkylsulfonyl oder C₁-C₃-Halogen- alkylsulfonyl, oder
- A: steht für einen heteroaromatischen Rest ausgewählt aus folgender Gruppe: Furan-2-yl, Furan- 3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Py- razol-4-yl, Imidazol-1-yl, Imidazol-2-yl, Imidazol-4-yl, Pyridin-2-yl, Pyridin-3-yl, Pyridin-4- yl, Pyridazin-3-yl, Pyridazin-4-yl, Pyrimidin-2-yl, Pyrimidin-4-yl oder Pyrimidin-5-yl, der bis zu drei Substituenten enthalten kann, wobei die Substituenten unabhängig voneinan- der ausgewählt sind aus folgender Liste: Substituenten am Kohlenstoff: Cyano, Nitro, Halogen, C₁-C₄-Alkyl, C₁-C₃-Halogenalkyl, Cyclopropyl, C₁-C₄-Alkoxy, C₁-C₃-Halogenalkoxy, C₁-C₄-Alkylthio oder C₁-C₃-Halogen- alkylthio, Substituenten am Stickstoff: C₁-C₄-Alkyl, C₁-C₃-Halogenalkyl, Cyclopropyl, C₂-C₄-Alkenyl oder C₂-C₄-Alkinyl,
- G: steht für C(R⁵)₂,
- Y: steht für Schwefel oder Sauerstoff,
- R¹: steht für Wasserstoff, C₁-C₂-Alkyl oder Halogen,
- n =: 0 bis 2,
- R²: steht für C₁-C₆-Alkyl, C₁-C₂-Halogenalkyl, C₂-C₆-Alkenyl, C₂-C₄-Alkynyl, Adamantan-1-yl oder Adamantan-2-yl, oder
- R²: steht für C₅-C₁₀-Cycloalkyl, das bis zu vier Substituenten enthalten kann, wobei die Substituenten unabhängig voneinander ausgewählt sind aus folgender Liste: Cyano, Halogen, C₁-C₄-Alkyl, C₁-C₃-Halogenalkyl, C₃-C₆-Cycloalkyl, C₂-C₄-Alkenyl, C₂-C₄- Halogenalkenyl, C₂-C₄-Alkinyl, C₂-C₄-Halogenalkinyl, Tri(C₁-C₃-alkyl)silyl, Phenyl, Oxo, C₁-C₄-Alkoxy, C₁-C₃-Halogenalkoxy, C₂-C₄-Alkenyloxy, C₂-C₄-Alkinyloxy, C₁-C₄-Alkylthio oder C₁-C₃-Halogenalkylthio, oder
- R²: steht für C₅-C₁₀-Cycloalkenyl, das bis zu vier Substituenten enthalten kann, wobei die Substituenten unabhängig voneinander ausgewählt sind aus folgender Liste: Cyano, Halogen, C₁-C₄-Alkyl, C₁-C₃-Halogenalkyl, C₃-C₆-Cycloalkyl, C₂-C₄-Alkenyl, C₂-C₄- Halogenalkenyl, C₂-C₄-Alkinyl, C₂-C₄-Halogenalkinyl, Tri(C₁-C₃-alkyl)silyl, Phenyl, Oxo, C₁-C₄-Alkoxy, C₁-C₃-Halogenalkoxy, C₂-C₄-Alkenyloxy, C₂-C₄-Alkinyloxy, C₁-C₄-Alkylthio oder C₁-C₃-Halogenalkylthio, oder
- R²: steht für Phenyl, das bis zu drei Substituenten enthalten kann, wobei die Substituenten unabhängig voneinander ausgewählt sind aus folgender Liste: Cyano, Nitro, Halogen, C₁-C₄-Alkyl, C₁-C₃-Halogenalkyl, C₃-C₆-Cycloalkyl, C₂-C₄-Alkenyl, C₂-C₄-Halogenalkenyl, C₂-C₄-Alkinyl, C₂-C₄-Halogenalkinyl, Tri(C₁-C₃-alkyl)silyl, Benzyl, Phenyl, C₁-C₄-Alkoxy, C₁-C₃-Halogenalkoxy, C₂-C₄-Alkenyloxy, C₂-C₄-Alkinyloxy, C₁-C₄- Alkylthio, C₁-C₃-Halogenalkylthio, (C₁-C₄-Alkoxy)carbonyl, (C₁-C₄-Alkyl)carbonyl, C₁-C₄- Alkylsulfinyl, C₁-C₃-Halogenalkylsulfinyl, C₁-C₄-Alkylsulfonyl oder C₁-C₃- Halogenalkylsulfonyl, oder
- R²: steht für Naphthalen-1-yl, Naphthalen-2-yl, 1,2,3,4-Tetrahydronaphthalen-1-yl, 1,2,3,4- Tetrahydronaphthalen-2-yl, 5,6,7,8-Tetrahydronaphthalen-1-yl, 5,6,7,8-Tetrahydronaph- thalen-2-yl, Decalin-1-yl, Decalin-2-yl, 1H-Inden-1-yl, 1H-Inden-2-yl, 1H-Inden-3-yl, 1H- Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl, das bis zu drei Substituenten enthalten kann, wobei die Substituenten unabhängig voneinan- der ausgewählt sind aus folgender Liste: Cyano, Nitro, Halogen, C₁-C₄-Alkyl, C₁-C₃-Halogenalkyl, C₃-C₆-Cycloalkyl, C₂-C₄-Alkenyl, C₂-C₄-Halogenalkenyl, C₂-C₄-Alkinyl, C₂-C₄-Halogenalkinyl, Tri(C₁-C₃-alkyl)silyl, Benzyl, Phenyl, C₁-C₄-Alkoxy, C₁-C₃-Halogenalkoxy, C₂-C₄-Alkenyloxy, C₂-C₄-Alkinyloxy, C₁-C₄- Alkylthio oder C₁-C₃-Halogenalkylthio, oder
- R²: steht für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Py- razol-1-yl, Pyrazol-3-yl, Pyrazol-4-yl, Imidazol-1-yl, Imidazol-2-yl, Imidazol-4-yl, 1,2,4- Oxadiazol-3-yl, 1,2,4-Oxadiazol-5-yl, 1,3,4-Oxadiazol-2-yl, Tetrazol-5-yl, 1,2,4-Thiadiazol- 3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Tetrazol-5-yl, Pyridin-2-yl, Pyridin-3-yl, Pyridin-4-yl, Pyridazin-3-yl, Pyridazin-4-yl, Pyrimidin-2-yl, Py- rimidin-4-yl, Pyrimidin-5-yl, Pyrazin-2-yl, 1,3,5-Triazin-2-yl oder 1,2,4-Triazin-3-yl, das bis zu drei Substituenten enthalten kann, wobei die Substituenten unabhängig voneinan- der ausgewählt sind aus folgender Liste: Substituenten am Kohlenstoff: Cyano, Nitro, Halogen, C₁-C₄-Alkyl, C₁-C₃-Halogenalkyl, C₃- C₆-Cycloalkyl, C₂-C₄-Alkenyl, C₂-C₄-Halogenalkenyl, C₂-C₄-Alkinyl, C₂-C₄-Halogenalkinyl, Tri(C₁-C₃-alkyl)silyl, Benzyl, Phenyl, C₁-C₄-Alkoxy, C₁-C₃-Halogenalkoxy, C₁-C₄-Alkylthio, C₁-C₃-Halogenalkylthio, (C₁-C₄-Alkoxy)carbonyl, (C₁-C₄-Alkyl)carbonyl, (C₁-C₃-Halogen- alkyl)carbonyl, (C₁-C₄-Alkyl)carbonyloxy, (C₁-C₄-Alkyl)carbonylthio, C₁-C₄-Alkylsulfinyl, C₁-C₃-Halogenalkylsulfinyl, C₁-C₄-Alkylsulfonyl oder C₁-C₃-Halogenalkylsulfonyl, Substituenten am Stickstoff: C₁-C₄-Alkyl, C₁-C₃-Halogenalkyl, C₃-C₆-Cycloalkyl, C₂-C₄- Alkenyl, C₂-C₄-Halogenalkenyl, C₂-C₄-Alkinyl, C₂-C₄-Halogenalkinyl oder Phenyl, oder
- R²: steht für Indol-1-yl, Indol-2-yl, Indol-3-yl, Indol-4-yl, Indol-5-yl, Indol-6-yl, Indol-7-yl, Ben- zimidazol-1-yl, Benzimidazol-2-yl, Benzimidazol-4-yl, Benzimidazol-5-yl, Indazol-1-yl, In- dazol-3-yl, Indazol-4-yl, Indazol-5-yl, Indazol-6-yl, Indazol-7-yl, Indazol-2-yl, 1-Ben- zofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1- Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3- Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochino- lin-5-yl, Isochinolin-6-yl, Isochinolin-7-yl oder Isochinolin-8-yl, das bis zu drei Substituenten enthalten kann, wobei die Substituenten unabhängig voneinan- der ausgewählt sind aus folgender Liste: Substituenten am Kohlenstoff: Cyano, Nitro, Halogen, C₁-C₄-Alkyl, C₁-C₃-Halogenalkyl, C₃- C₆-Cycloalkyl, C₂-C₄-Alkenyl, C₂-C₄-Halogenalkenyl, C₂-C₄-Alkinyl, C₂-C₄-Halogenalkinyl, Tri(C₁-C₃-alkyl)silyl, Benzyl, Phenyl, C₁-C₄-Alkoxy, C₁-C₃-Halogenalkoxy, C₁-C₄-Alkylthio, C₁-C₃-Halogenalkylthio, (C₁-C₄-Alkoxy)carbonyl, (C₁-C₄-Alkyl)carbonyl, (C₁-C₃-Halogen- alkyl)carbonyl, (C₁-C₄-Alkyl)carbonyloxy, (C₁-C₄-Alkyl)carbonylthio, C₁-C₄-Alkylsulfinyl, C₁-C₃-Halogenalkylsulfinyl, C₁-C₄-Alkylsulfonyl oder C₁-C₃-Halogenalkylsulfonyl, Substituenten am Stickstoff: C₁-C₄-Alkyl, C₁-C₃-Halogenalkyl, C₃-C₆-Cycloalkyl, C₂-C₄- Alkenyl, C₂-C₄-Halogenalkenyl, C₂-C₄-Alkinyl, C₂-C₄-Halogenalkinyl oder Phenyl, oder
- R²: steht für C₅-C₁₀-Heterocyclyl, das bis zu drei Substituenten enthalten kann, wobei mögliche Substituenten unabhängig voneinander ausgewählt sind aus Folgender Liste: Substituenten am Kohlenstoff: Cyano, Nitro, Halogen, C₁-C₄-Alkyl, C₁-C₃-Halogenalkyl, C₃- C₆-Cycloalkyl, C₂-C₄-Alkenyl, C₂-C₄-Halogenalkenyl, C₂-C₄-Alkinyl, C₂-C₄-Halogenalkinyl, Tri(C₁-C₃-alkyl)silyl, Benzyl, Phenyl, C₁-C₄-Alkoxy, C₁-C₃-Halogenalkoxy, C₁-C₄-Alkylthio, C₁-C₃-Halogenalkylthio, (C₁-C₄-Alkoxy)carbonyl, (C₁-C₄-Alkyl)carbonyl, (C₁-C₃-Halogen- alkyl)carbonyl, (C₁-C₄-Alkyl)carbonyloxy, (C₁-C₄-Alkyl)carbonylthio, C₁-C₄-Alkylsulfinyl, C₁-C₃-Halogenalkylsulfinyl, C₁-C₄-Alkylsulfonyl oder C₁-C₃-Halogenalkylsulfonyl, Substituenten am Stickstoff: C₁-C₄-Alkyl, C₁-C₃-Halogenalkyl, C₃-C₆-Cycloalkyl, C₂-C₄- Alkenyl, C₂-C₄-Halogenalkenyl, C₂-C₄-Alkinyl, C₂-C₄-Halogenalkinyl oder Phenyl,
- R⁵: ist gleich oder verschieden unabhängig voneinander Wasserstoff, Methyl, Ethyl oder CF₃,

sowie agrochemisch wirksame Salze davon,

Besonders bevorzugt werden Verbindungen der Formel (I), in denen ein oder mehrere der Symbole eine der folgenden Bedeutungen haben:
- A: steht für Phenyl, das bis zu zwei Substituenten enthalten kann, wobei die Substituenten unabhängig voneinander ausgewählt sind aus folgender Liste: Cyano, Nitro, Halogen, Methyl, Ethyl, Propyl, 1-Methylethyl, 1,1-Dimethylethyl, CF₃, CHF₂, C₂F₅, CCl₃, OMe, OEt, O*iso*Pr, OCF₃, OCHF₂, OC₂F₅, SMe oder SCF₃, oder
- A: steht für einen heteroaromatischen Rest ausgewählt aus folgender Gruppe: Furan-2-yl, Furan- 3-yl, Thiophen-2-yl, Thiophen-3-yl, Oxazol-2-yl, Oxazol-4-yl, Oxazol-5-yl, Thiazol-2-yl, Thiazol-4-yl, Thiazol-5-yl, Pyrazol-1-yl, Pyrazol-3-yl, Pyrazol-4-yl, Imidazol-1-yl, Imidazol- 2-yl, Imidazol-4-yl, Pyridin-2-yl, Pyridin-3-yl oder Pyridin-4-yl, der bis zu zwei Substituenten enthalten kann, wobei die Substituenten unabhängig voneinan- der ausgewählt sind aus folgender Liste: Substituenten am Kohlenstoff: Cyano, Halogen, Methyl, Ethyl, Propyl, 1-Methylethyl, 1,1- Dimethylethyl, CF₃, CHF₂, C₂F₅, CCl₃, Cyclopropyl, OMe, OEt, O*iso*Pr, OCF₃, OCHF₂, OC₂F₅, SMe oder SCF₃, Substituenten am Stickstoff: Methyl, Ethyl, Propyl, 1-Methylethyl, 1,1-Dimethylethyl, CF₃, CHF₂, C₂F₅ oder CCl₃,
- G: steht für CH₂,
- Y: steht für Schwefel oder Sauerstoff,
- R¹: steht für Wasserstoff, Methyl, Chlor oder Brom,
- n =: 0 bis 2,
- R²: steht für C₁-C₆-Alkyl, C₁-C₂-Halogenalkyl, Adamantan-1-yl oder Adamantan-2-yl, oder
- R²: steht für C₅-C₈-Cycloalkyl, das bis zu vier Substituenten enthalten kann, wobei die Substituenten unabhängig voneinander ausgewählt sind aus folgender Liste: Cyano, Halogen, Methyl, Ethyl, Propyl, 1-Methylethyl, 1,1-Dimethylethyl, CF₃, CHF₂, C₂F₅, CCl₃, -CH=CH₂, -CH₂CH=CH₂, Tri(methyl)silyl, Phenyl, Oxo, OMe, OEt, O*iso*Pr, OCF₃, OCHF₂, OC₂F₅, SMe oder SCF₃, oder
- R²: steht für C₅-C₈-Cycloalkenyl, das bis zu vier Substituenten enthalten kann, wobei die Substituenten unabhängig voneinander ausgewählt sind aus folgender Liste: Cyano, Halogen, Methyl, Ethyl, Propyl, 1-Methylethyl, 1,1-Dimethylethyl, CF₃, CHF₂, C₂F₅, CCl₃, -CH=CH₂, -CH₂CH=CH₂, Tri(methyl)silyl, Phenyl, Oxo, OMe, OEt, O*iso*Pr, OCF₃, OCHF₂, OC₂F₅, SMe oder SCF₃, oder
- R²: steht für Phenyl, das bis zu drei Substituenten enthalten kann, wobei die Substituenten unabhängig voneinander ausgewählt sind aus folgender Liste: Cyano, Nitro, Halogen, Methyl, Ethyl, 1-Methylethyl, 1,1-Dimethylethyl, CF₃, CHF₂, C₂F₅, CCl₃, -CH=CH₂, -CH₂CH=CH₂, -C≡CH, Tri(methyl)silyl, Benzyl, Phenyl, OMe, OEt, Oi- *so*Pr, OCF₃, OCHF₂, OC₂F₅, SMe oder SCF₃, oder
- R²: steht für Naphthalen-1-yl, Naphthalen-2-yl, 1,2,3,4-Tetrahydronaphthalen-1-yl, 1,2,3,4-Te- trahydronaphthalen-2-yl, 5,6,7,8-Tetrahydronaphthalen-1-yl, 5,6,7,8-Tetrahydronaphthalen- 2-yl, Decalin-1-yl, Decalin-2-yl, 1H-Inden-1-yl, 1H-Inden-2-yl, 1H-Inden-3-yl, 1H-Inden-4- yl, 1H-Inden-5-yl, 1H-Inden-6-yl, 1H-Inden-7-yl, Indan-1-yl, Indan-2-yl, Indan-3-yl, Indan- 4-yl oder Indan-5-yl, das bis zu drei Substituenten enthalten kann, wobei die Substituenten unabhängig voneinan- der ausgewählt sind aus folgender Liste: Cyano, Nitro, Halogen, Methyl, Ethyl, 1-Methylethyl, 1,1-Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃, oder
- R²: steht für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Py- razol-1-yl, Pyrazol-3-yl, Pyrazol-4-yl, Imidazol-1-yl, Imidazol-2-yl, Imidazol-4-yl, 1,2,4- Oxadiazol-3-yl, 1,2,4-Oxadiazol-5-yl, 1,3,4-Oxadiazol-2-yl, Tetrazol-5-yl, 1,2,4-Thiadiazol- 3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Pyridin-2-yl, Py- ridin-3-yl, Pyridin-4-yl, Pyridazin-3-yl, Pyridazin-4-yl, Pyrimidin-2-yl, Pyrimidin-4-yl, Py- rimidin-5-yl, Pyrazin-2-yl, 1,3,5-Triazin-2-yl oder 1,2,4-Triazin-3-yl, das bis zu drei Substituenten enthalten kann, wobei die Substituenten unabhängig voneinan- der ausgewählt sind aus folgender Liste: Substituenten am Kohlenstoff: Cyano, Nitro, Halogen, Methyl, Ethyl, 1-Methylethyl, 1,1- Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃, Substituenten am Stickstoff: Methyl, Ethyl, 1-Methylethyl, 1,1-Dimethylethyl oder Phenyl, oder
- R²: steht für Indol-1-yl, Indol-2-yl, Indol-3-yl, Indol-4-yl, Indol-5-yl, Indol-6-yl, Indol-7-yl, Ben- zimidazol-1-yl, Benzimidazol-2-yl, Benzimidazol-4-yl, Benzimidazol-5-yl, Indazol-1-yl, In- dazol-3-yl, Indazol-4-yl, Indazol-5-yl, Indazol-6-yl, Indazol-7-yl, Indazol-2-yl, 1- Benzofuran-2-yl, 1-Benzofuran-3-yl, 1-Benzofuran-4-yl, 1-Benzofuran-5-yl, 1-Benzofuran- 6-yl, 1-Benzofuran-7-yl, 1-Benzothiophen-2-yl, 1-Benzothiophen-3-yl, 1-Benzothiophen-4- yl, 1-Benzothiophen-5-yl, 1-Benzothiophen-6-yl, 1-Benzothiophen-7-yl, 1,3-Benzothiazol-2- yl, 1,3-Benzothiazol-4-yl, 1,3-Benzothiazol-5-yl, 1,3-Benzothiazol-6-yl, 1,3-Benzothiazol-7- yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, Chinolin-2-yl, Chinolin-3-yl, Chinolin-4-yl, Chinolin-5-yl, Chinolin-6- yl, Chinolin-7-yl, Chinolin-8-yl, Isochinolin-1-yl, Isochinolin-3-yl, Isochinolin-4-yl, Isochi- nolin-5-yl, Isochinolin-6-yl, Isochinolin-7-yl oder Isochinolin-8-yl, das bis zu drei Substituenten enthalten kann, wobei die Substituenten unabhängig voneinan- der ausgewählt sind aus folgender Liste: Substituenten am Kohlenstoff: Cyano, Nitro, Halogen, Methyl, Ethyl, 1-Methylethyl, 1,1- Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃, Substituenten am Stickstoff: Methyl, Ethyl, 1-Methylethyl, 1,1-Dimethylethyl oder Phenyl, oder
- R²: steht für C₅-C₁₀-Heterocyclyl, das bis zu drei Substituenten enthalten kann, wobei mögliche Substituenten unabhängig voneinander ausgewählt sind aus folgender Liste: Substituenten am Kohlenstoff: Cyano, Nitro, Halogen, Methyl, Ethyl, 1-Methylethyl, 1,1- Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃, Substituenten am Stickstoff: Methyl, Ethyl, 1-Methylethyl, 1,1-Dimethylethyl oder Phenyl,

sowie agrochemisch wirksame Salze davon,

Ganz besonders bevorzugt werden Verbindungen der Formel (I), in denen ein oder mehrere der Symbole eine der folgenden Bedeutungen haben:
- A: steht für Phenyl, das bis zu zwei Substituenten enthalten kann, wobei die Substituenten unabhängig voneinander ausgewählt sind aus folgender Liste: Cyano, Halogen, Methyl, Ethyl, Propyl, 1-Methylethyl, 1,1-Dimethylethyl, CF₃, CHF₂, C₂F₅, CCl₃, OMe, OCF₃, OCHF₂ oder OC₂F₅, oder
- A: steht für einen heteroaromatischen Rest ausgewählt aus folgender Gruppe: Pyrazol-1-yl, Py- razol-3-yl, Pyrazol-4-yl, Pyridin-2-yl, Pyridin-3-yl oder Pyridin-4-yl, der bis zu zwei Substituenten enthalten kann, wobei die Substituenten unabhängig voneinan- der ausgewählt sind aus folgender Liste: Substituenten am Kohlenstoff: Cyano, Halogen, Methyl, Ethyl, Propyl, 1-Methylethyl, 1,1- Dimethylethyl, CF₃, CHF₂, C₂F₅, CCl₃, OMe, OCF₃, OCHF₂ oder OC₂F₅, Substituenten am Stickstoff: Methyl, Ethyl oder CF₃,
- G: steht für CH₂,
- Y: steht für Schwefel oder Sauerstoff,
- R¹: steht für Wasserstoff,
- n =: 0 bis 2,
- R²: steht für Methyl, Ethyl, 1-Methylethyl, 1,1-Dimethylethyl, Propyl, Butyl, Pentyl, Hexyl, A- damantan-1-yl oder Adamantan-2-yl, oder
- R²: steht für C₅-C₈-Cycloalkyl, das bis zu vier Substituenten enthalten kann, wobei die Substituenten unabhängig voneinander ausgewählt sind aus folgender Liste: Methyl, Ethyl, 1-Methylethyl oder 1,1-Dimethylethyl, oder
- R²: steht für C₅-C₈-Cycloalkenyl, das bis zu vier Substituenten enthalten kann, wobei die Substituenten unabhängig voneinander ausgewählt sind aus folgender Liste: Methyl, Ethyl, 1-Methylethyl oder 1,1-Dimethylethyl, oder
- R²: steht für Phenyl, das bis zu zwei Substituenten enthalten kann, wobei die Substituenten unabhängig voneinander ausgewählt sind aus folgender Liste: Cyano, Nitro, Halogen, Methyl, Ethyl, 1-Methylethyl, 1,1-Dimethylethyl, CF₃, CHF₂, C₂F₅, CCl₃, -CH=CH₂, -CH₂CH=CH₂, Benzyl, Phenyl, OMe, OCF₃, OCHF₂, OC₂F₅, SMe oder SCF₃, oder
- R²: steht für Naphthalen-1-yl, Naphthalen-2-yl, 1,2,3,4-Tetrahydronaphthalen-1-yl, 1,2,3,4- Tetrahydronaphthalen-2-yl, 5,6,7,8-Tetrahydronaphthalen-1-yl, 5,6,7,8-Tetrahydronaph- thalen-2-yl, Decalin-1-yl, Decalin-2-yl, 1H-Inden-1-yl, 1H-Inden-2-yl, 1H-Inden-3-yl, 1H- Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl, das bis zu zwei Substituenten enthalten kann, wobei die Substituenten unabhängig voneinan- der ausgewählt sind aus folgender Liste: Cyano, Nitro, Halogen, Methyl, Ethyl, 1-Methylethyl, 1,1-Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃, oder
- R²: steht für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Py- razol-1-yl, Pyrazol-3-yl, Pyrazol-4-yl, Imidazol-1-yl, Imidazol-2-yl, Imidazol-4-yl, 1,2,4- Oxadiazol-3-yl, 1,2,4-Oxadiazol-5-yl, 1,3,4-Oxadiazol-2-yl, Tetrazol-5-yl, 1,2,4-Thiadiazol- 3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Pyridin-2-yl, Py- ridin-3-yl, Pyridin-4-yl, Pyridazin-3-yl, Pyridazin-4-yl, Pyrimidin-2-yl, Pyrimidin-4-yl, Py- rimidin-5-yl, Pyrazin-2-yl, 1,3,5-Triazin-2-yl oder 1,2,4-Triazin-3-yl, das bis zu zwei Substituenten enthalten kann, wobei die Substituenten unabhängig voneinan- der ausgewählt sind aus folgender Liste: Substituenten am Kohlenstoff: Cyano, Nitro, Halogen, Methyl, Ethyl, 1-Methylethyl, 1,1- Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃, Substituenten am Stickstoff: Methyl, Ethyl, 1-Methylethyl, 1,1-Dimethylethyl oder Phenyl, oder
- R²: steht für Indol-1-yl, Indol-2-yl, Indol-3-yl, Indol-4-yl, Indol-5-yl, Indol-6-yl, Indol-7-yl, Ben- zimidazol-1-yl, Benzimidazol-2-yl, Benzimidazol-4-yl, Benzimidazol-5-yl, Indazol-1-yl, In- dazol-3-yl, Indazol-4-yl, Indazol-5-yl, Indazol-6-yl, Indazol-7-yl, Indazol-2-yl, 1-Benzo- furan-2-yl, 1-Benzofuran-3-yl, 1-Benzofuran-4-yl, 1-Benzofuran-5-yl, 1-Benzofuran-6-yl, 1- Benzofuran-7-yl, 1-Benzothiophen-2-yl, 1-Benzothiophen-3-yl, 1-Benzothiophen-4-yl, 1- Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3- Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochino- lin-5-yl, Isochinolin-6-yl, Isochinolin-7-yl oder Isochinolin-8-yl, das bis zu zwei Substituenten enthalten kann, wobei die Substituenten unabhängig voneinan- der ausgewählt sind aus folgender Liste: Substituenten am Kohlenstoff: Cyano, Nitro, Halogen, Methyl, Ethyl, 1-Methylethyl, 1,1- Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃, Substituenten am Stickstoff: Methyl, Ethyl, 1-Methylethyl, 1,1-Dimethylethyl oder Phenyl, oder
- R²: steht für Piperidin-1-yl, Piperidin-2-yl, Piperidin-3-yl, Piperidin-4-yl, Piperazin-1-yl, Pipera- zin-2-yl, Piperazin-3-yl, Morpholin-1-yl, Morpholin-2-yl, Morpholin-3-yl, Tetrahydropyran- 2-yl, Tetrahydropyran-3-yl, Tetrahydropyran-4-yl, 1,2,3,4-Tetrahydrochinolin-1-yl, 1,2,3,4- tetrahydroisochinolin-2-yl, 1,2,3,4-Tetrahydrochinoxalin-1-yl, Indolin-1-yl, Isoindolin-2-yl, Decahydrochinolin-1-yl oder Decahydroisochinolin-2-yl, das bis zu zwei Substituenten enthalten kann, wobei mögliche Substituenten unabhängig voneinander ausgewählt sind aus folgender Liste: Substituenten am Kohlenstoff: Cyano, Nitro, Halogen, Methyl, Ethyl, 1-Methylethyl, 1,1- Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃, Substituenten am Stickstoff: Methyl, Ethyl, 1-Methylethyl, 1,1-Dimethylethyl oder Phenyl,
sowie agrochemisch wirksame Salze davon,

Insbesondere bevorzugt werden Verbindungen der Formel (I), in denen ein oder mehrere der Symbole eine der folgenden Bedeutungen haben:
- A: steht für 5-Methyl-3-(trifluormethyl)-1H-pyrazol-1-yl,
- G: steht für CH₂,
- Y: steht für Schwefel oder Sauerstoff,
- R¹: steht für Wasserstoff,
- n =: 0 bis 2,
- R²: steht für Naphthalen-1-yl, Adamantan-1-yl, Cyclohexyl, 1-Phenyl-tetrazol-5-yl oder Chino- lin-8-yl
sowie agrochemisch wirksame Salze davon,

Weiterhin insbesondere bevorzugt werden Verbindungen der Formel **(I),** in denen
- A: für ein unsubstituiertes oder substituiertes Phenyl steht,
wobei die Substituenten unabhängig voneinander ausgewählt sind aus folgender Liste:
Cyano, Halogen, Methyl, Ethyl, Propyl, 1-Methylethyl, 1,1-Dimethylethyl, CF₃, CHF₂, C₂F₅, CCl₃, OMe, OCF₃, OCHF₂ oder OC₂F₅,
und G steht für -CH₂-,
wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
- A: für ein unsubstituiertes oder substituiertes Pyrazol-1-yl oder Pyrazol-4-yl steht,
das bis zu zwei Substituenten enthalten kann, wobei die Substituenten unabhängig voneinander ausgewählt sind aus folgender Liste:
Substituenten am Kohlenstoff: Cyano, Halogen, Methyl, Ethyl, Propyl, 1-Methylethyl, 1,1-Dimethylethyl, CF₃, CHF₂, C₂F₅, CCl₃, OMe, OCF₃, OCHF₂ oder OC₂F₅,
Substituenten am Stickstoff: Methyl, Ethyl oder CF₃, und G steht für -CH₂-,
   wobei die übrigen Substituenten eine oder mehrere der oben genannten Bedeutungen haben,
   sowie die agrochemisch wirksamen Salzen davon,

Weiterhin insbesondere bevorzugt werden Verbindungen der Formel **(I),** in denen
- A: für 5-Methyl-3-(trifluormethyl)-1H-pyrazol-1-yl steht, wobei die übrigen Substituenten eine oder mehrere der oben genannten Bedeutungen haben, sowie die agrochemisch wirksamen Salzen davon,

Weiterhin insbesondere bevorzugt werden Verbindungen der Formel **(I),** in denen
- G: für -CH₂- steht,
wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
- Y: für Sauerstoff steht,
wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
- Y: für Schwefel steht,
wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
- R¹: für Wasserstoff steht,
wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
n=0,
wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
n = 1,
wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
n=2,
wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
- R²: für Naphthalen-1-yl steht,

wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
- R²: für Adamantan-1-yl steht,
wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
- R²: für Cyclohexyl steht,
wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
- R²: für 1-Phenyl-tetrazol-5-yl steht,
wobei die übrigen Substituenten eine oder mehrere der oben genannten Bedeutungen haben,
sowie die agrochemisch wirksamen Salzen davon,

Weiterhin insbesondere bevorzugt werden Verbindungen der Formel **(I),** in denen
- R²: für Chinolin-8-yl steht,
wobei die übrigen Substituenten eine oder mehrere der oben genannten Bedeutungen haben,
sowie die agrochemisch wirksamen Salzen davon,

Die zuvor genannten Reste-Definitionen können untereinander in beliebiger Weise kombiniert werden. Außerdem können einzelne Definitionen entfallen.

Je nach Art der oben definierten Substituenten weisen die Verbindungen der Formel **(I)** saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel (**I**) Amino, Alkylamino oder andere, basische Eigenschaften induzierende Gruppen, so können diese Verbindungen mit Säuren zu Salzen umgesetzt werden oder fallen durch die Synthese direkt als Salze an. Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₄-Alkyl-Gruppen, Mono-, Di- und Trialkanolamine von C₁-C₄-Alkanolen, Cholin sowie Chlorcholin.

Die so erhältlichen Salze weisen ebenfalls fungizide, herbizide und insektizide Eigenschaften auf.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, gesättigte oder einfach oder doppelt ungesättigte C₆-C₂₀-Fettsäuren, Alkylschwefelsäuremonoester, Alkylsulfonsäuren (Sulfonsäuren mit geradkettigen oder verzweigten Alkylresten mit 1 bis 20 Kohlenstoffatomen), Arylsulfonsäuren oder Aryldisulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder Aryldiphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc..

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Gegebenenfalls substituierte Gruppen können einfach oder mehrfach substituiert sein, wobei bei Mehrfachsubstitutionen die Substituenten gleich oder verschieden sein können.

Bei den in den vorstehenden Formeln angegebenen Definitionen der Symbole wurden Sammelbegriffe verwendet, die allgemein repräsentativ für die folgenden Substituenten stehen:

### Halogen: Fluor, Chlor, Brom und Iod;

**Alkyl:** gesättigte, geradkettige oder verzweigte Kohlenwasserstoffreste mit 1 bis 8 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;

**Alkenyl:** ungesättigte, geradkettige oder verzweigte Kohlenwasserstoffreste mit 2 bis 8 Kohlenstoffatomen und einer Doppelbindung in einer beliebigen Position, z.B. (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;

**Alkenyl:** geradkettige oder verzweigte Kohlenwasserstoffgruppen mit 2 bis 8 Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;

**Alkoxy:** gesättigte, geradkettige oder verzweigte Alkoxyreste mit 1 bis 8 Kohlenstoffatomen, z.B. (aber nicht beschränkt auf) C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;

**Alkylthio:** gesättigte, geradkettige oder verzweigte Alkylthioreste mit 1 bis 8 Kohlenstoffatomen, z.B. (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;

**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist;

**Alkylsulfinyl:** gesättigte, geradkettige oder verzweigte Alkylsulfinylreste mit 1 bis 8 Kohlenstoffatomen, z.B. (aber nicht beschränkt auf) C₁-C₆-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Di-methylpropylsulfinyl, 1-Ethylpropylsulfinyl, Hexylsulfinyl, 1,1-Dimethylpropylsulfinyl, 1,2-Dimethylpropylsulfinyl,1-Methylpentylsulfinyl, 2-Methylpentyl-sulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;

**Alkylsulfonyl:** gesättigte, geradkettige oder verzweigte Alkylsulfonylreste mit 1 bis 8 Kohlenstoffatomen, z.B. (aber nicht beschränkt auf) C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Di-methylpropylsulfonyl, 1-Ethylpropylsulfonyl, Hexylsulfonyl, 1,1-Dimethylpropylsulfonyl, 1,2-Dimethylpropylsulfonyl,1-Methylpentylsulfonyl, 2-Methylpentyl-sulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;

**Cycloalkyl:** monocyclische, gesättigte Kohlenwasserstoffgruppen mit 3 bis 10 Kohlenstoffringgliedern, z.B. (aber nicht beschränkt auf) Cyclopropyl, Cyclopentyl und Cyclohexyl;

**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl;

**Halogenalkoxy:** geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy;

**Halogenalkylthio:** geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio;

**Heteroaryl:** 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;

**5-gliedriges Heteroaryl: enthaltend ein bis vier Stickstoffatome oder ein bis drei Stickstoffatome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. (aber nicht beschränkt auf) 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;

**über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, in der ein oder zwei C-Atome durch N-Atome ersetzt sein können, wobei diese Ringe über eines der Stickstoffringglieder an das Gerüst gebunden sind, z.B. (aber nicht beschränkt auf) 1-Pyrrolyl, 1-Pyrazolyl, 1,2,4-Triazol-1-yl, 1-Imidazolyl, 1,2,3-Triazol-1-yl und 1,3,4-Triazol-1-yl;

**6-gliedriges Heteroaryl, enthaltend ein bis vier Stickstoffatome:** 6-Ring Heteroarylgruppen, welche neben Kohlenstoffatomen ein bis drei bzw. ein bis vier Stickstoffatome als Ringglieder enthalten können, beispielsweise (aber nicht beschränkt auf) 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;

**Benzokondensiertes 5-gliedriges Heteroaryl, enthaltend ein bis drei Stickstoffatome oder ein Stickstoffatom und ein Sauerstoff- oder Schwefelatom:** z.B. (aber nicht beschränkt auf) Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl und 1,3-Benzoxazol-7-yl,

**Benzokondensiertes 6-gliedriges Heteroaryl, enthaltend ein bis drei Stickstoffatome:** z.B. (aber nicht beschränkt auf) Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, und Isochinolin-8-yl;

**Heterocyclyl:** drei- bis fünfzehngliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydro-pyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;

**Abgangsgruppe:** S_{N}1 oder S_{N}2-Abgangsgruppe, beispielsweise Chlor, Brom, Iod, Alkylsulfonate (-OSO₂-Alkyl, z.B. -OSO₂CH₃, -OSO₂CF₃) oder Arylsulfonate (-OSO₂-Aryl, z.B. -OSO₂Ph, -OSO₂PhMe);

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Erläuterung der Verfahren und Zwischenprodukte

Die Thiazolylpiperidin Derivate der Formel (I) lassen sich auf unterschiedliche Weise herstellen. Im Folgenden sind die möglichen Verfahren zunächst schematisch dargestellt. Wenn nicht anders angegeben haben die angegebenen Reste die oben angegebenen Bedeutungen. 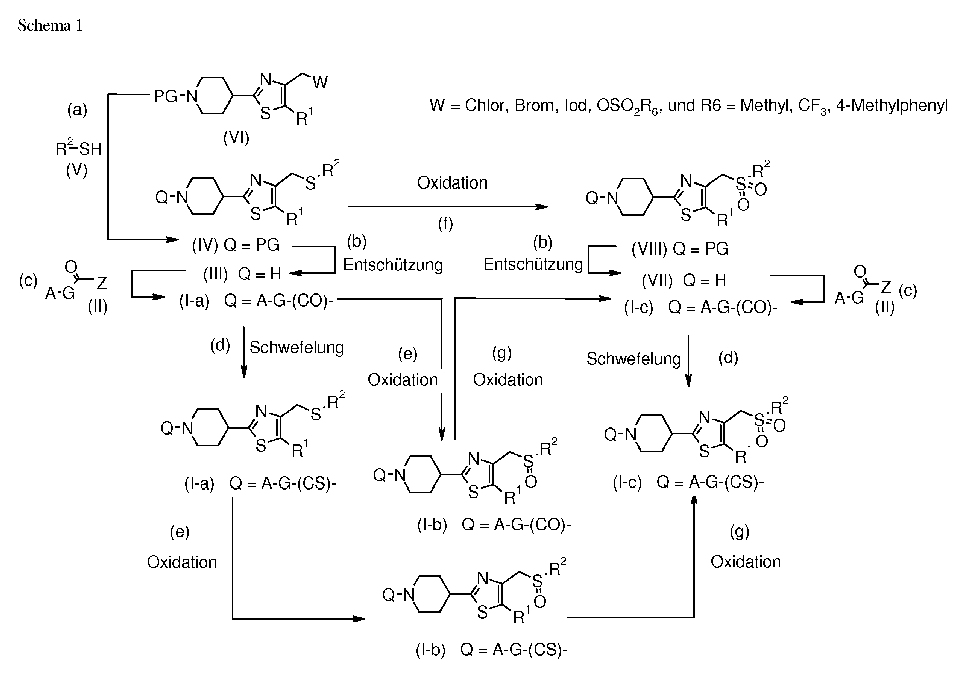 worin R¹, R², A und G die in Anspruch 1 angegebenen Bedeutungen haben.

Neu sind Verbindungen der Formel **(VI-a)** 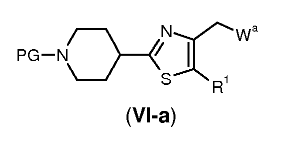 in denen die Symbole die folgenden Bedeutungen haben PG steht für Acetyl, C₁-C₄-Alkoxycarbonyl, Benzyl oder Benzyloxycarbonyl,
- W^{a} =: Brom oder Iod,
- R¹: hat die oben angegebenen allgemeinen, bevorzugten, besonders bevorzugten oder ganz be- sonders bevorzugten Bedeutungen,
sowie Salze davon,
zum Beispiel (**VI-a-1**):

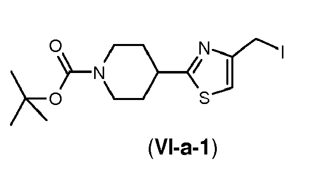

Neu sind Verbindungen der Formel **(IV)** 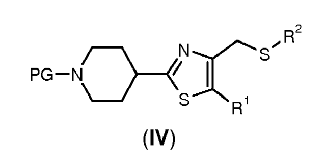 in denen die Symbole die folgenden Bedeutungen haben PG steht für Acetyl, C₁-C₄-Alkoxycarbonyl, Benzyl oder Benzyloxycarbonyl,
- R¹ und R²: haben die oben angegebenen allgemeinen, bevorzugten, besonders bevorzugten oder ganz besonders bevorzugten Bedeutungen,
sowie Salze davon,
zum Beispiel **(IV-1), (IV-2), (IV-3), (IV-4),** und **(IV-5):**

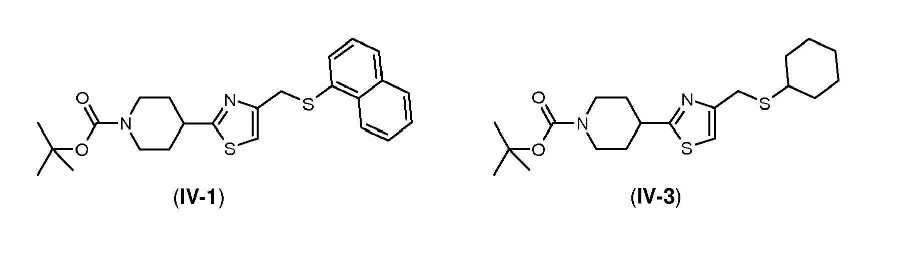

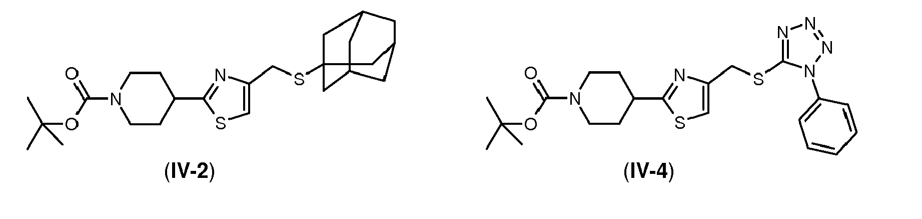

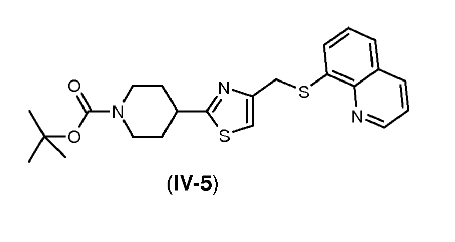

Ausgenommen ist die Verbindung tert-Butyl-4-[4-({[4-(methylsulfonyl)phenyl]sulfanyl}methyl)-1,3-thiazol-2-yl]piperidin-1-carboxylat.

Neu sind Verbindungen der Formel **(III)** 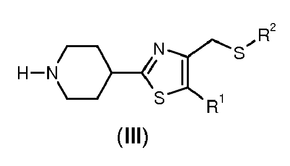 in denen die Symbole die folgenden Bedeutungen haben
- R¹ und R²: haben die oben angegebenen allgemeinen, bevorzugten, besonders bevorzugten oder ganz besonders bevorzugten Bedeutungen,
sowie Salze davon,

zum Beispiel **(111-1), (111-2), (111-3), (111-4),** und **(III-5),** 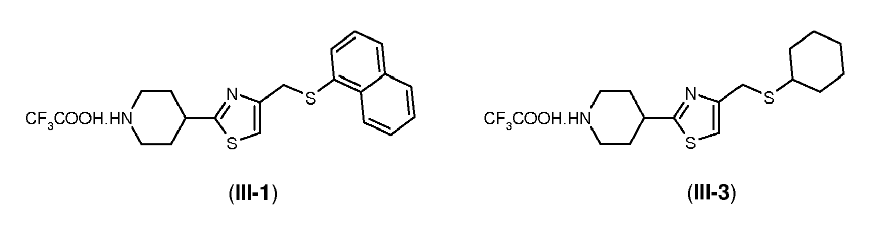 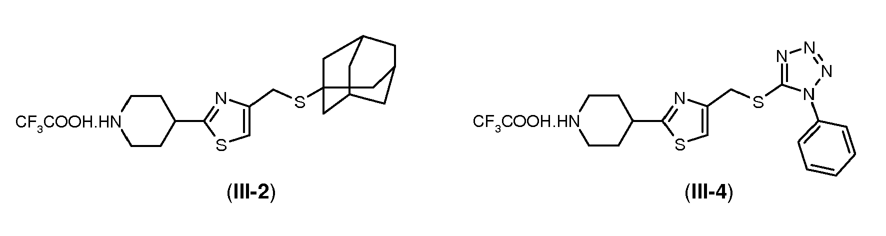 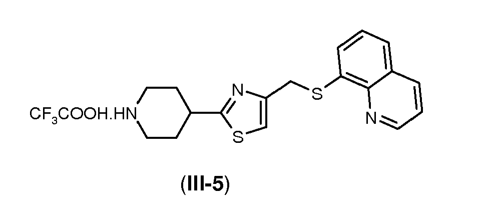 Eine Verbindung mit der allgemeinen Formel **(VI)** wird mit einer Verbindung der Formel **(V)** umgesetzt, um eine Verbindung mit der Formel **(IV)** zu erhalten (Schema 1). Die Schutzgruppe einer Verbindung mit der Formel **(IV)** oder **(VIII),** die mit PG gekennzeichnet ist, wird entfernt, so dass eine Verbindung mit der Formel **(III), (VII),** oder das entsprechende Salz entsteht (Schema 1). Diese Verbindungen können einer Kupplungsreaktion mit einem Substrat der Formel **(II)** unterzogen werden. Es entsteht eine Verbindung mit der Formel **(I-a)** oder **(I-c)** (Schema 1). Eine Verbindung mit der Formel **(I)** (Y = Sauerstoff) wird mit einem Schwefelungsreagenz versetzt, um eine Verbindung mit der Formel **(I)** (Y = Schwefel) zu generieren (Schema 1). Die Sulfide mit der allgemeinen Formel **(I-a),** werden mit einem Oxidationsmittel umgesetzt, um die Sulfoxide mit der Formel (**I-b**) zu erhalten (Schema 1). Die Oxidation die Sulfide **(I-a), (III)** oder **(IV)** kann auch bis zu den Sulfonen mit der allgemeinen Formel **(I-c), (VII)** oder **(VIII)** durchgeführt werden (Schema 1). Alternativ können die Sulfoxide **(I-b),** zu den Sulfonen mit der allgemeinen Formel **(I-c),** oxidiert werden (Schema 1).

### Schritt (a)

Eine Möglichkeit zur Synthese von Verbindungen der Formel **(IV)** ist in Schema 1 gezeigt.

Die Sulfide **(IV)** können durch einer nukleophile Substitution aus Verbindungen **(VI)** und Thiol **(V)** hergestellt werden (siehe z.B. J. Heterocylcic Chem. 1981, 18 (4), 789-793; Bioorg. Med. Chem. Lett. 2007, 17 (10), 2731-2734).

Verbindungen **(VI)** können durch Halogenierung (siehe z.B. WO 2008/013622; Eur. J. Org. Chem. 2007, 6, 934-942) oder Sulfonierung (siehe z.B. WO 2005/121130) der Alkohole **(IX)** hergestellt werden (Schema 2).

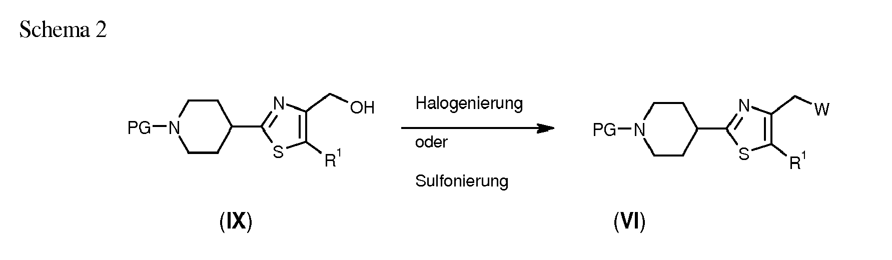

Die Alkohole **(IX)** sind bekannt oder können aus kommerziell erhältlichen Vorstufen nach literaturbeschriebenen Vorschriften hergestellt werden (siehe z.B. WO 2008/013925), zum Beispiel aus Ester der Formel **(X)** (kommerziell verfügbar). Eine bevorzugte Methode ist die Reduktion der Ester **(X)** mit z.B. Diisobutylaluminiumhydrid oder Lithiumaluminiumhydrid bei 0 °C - Raumtemperatur in einer Mischung bestehend aus Tetrahydrofuran.

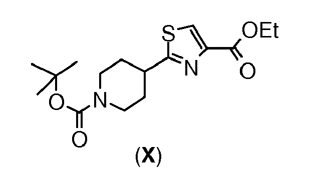

Halogenierung von **(IX)** kann mit einem Halogenierungreagenz, z.B. Thionylchlorid, Brom, Iod oder Carbontetrabromid, in Gegenwart eines Lösungsmittels, und gegebenenfalls Triphenylphospin und Imidazol durchgeführt werden. Das bevorzugte Lösungsmittel ist Dichlormethan. Die Reaktion wird normalerweise bei Temperaturen von 0 °C - 100 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einer halben Stunde und 48 Stunden. Sulfonierung von **(IX)** kann mit ein Alkyl- oder Arylsulfonylchlorid, z.B. Methylsulfonylchlorid und 4-Methylphenylsulfonylchlorid, in Gegenwart eines Lösungsmittels und einer Base, z.B. Triethylamin durchgeführt werden. Die bevorzugten Lösungsmittel sind Tetrahydrofuran und Dichlormethan. Die Reaktion wird normalerweise bei Temperaturen von 0 °C - 100 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einer halben Stunde und 48 Stunden. Nach Beendigung der Reaktion, werden die Verbindungen **(VI),** von der Reaktionsmischung durch eine der üblichen Trenntechniken getrennt. Falls notwendig werden die Verbindungen durch Destillation oder Chromatographie gereinigt oder können gegebenenfalls auch im nächsten Schritt ohne vorhergehende Reinigung eingesetzt werden.

Das Thiol **(V)** ist entweder kommerziell verfügbar oder nach literaturbekannten Methoden erhältlich (Beispiele, siehe z.B. "The Chemistry of Functional groups"; "The Chemistry of the Thiol Group"; John Wiley & Sons, 1974, 163-269, und darin zitierte Literatur).

Die nukleophile Substitution läuft ggf. in Gegenwart eines Lösungsmittels und einer geeigneten Base ab.

Als Lösungsmittel können alle üblichen, unter den Reaktionsbedingungen inerten Lösungsmittel, wie z.B. zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester), Amide (z.B. N,N-Dimethylformamid, N,N-Dimethylacetamid), Dimethylsulfoxid, 1,3-Dimethyl-2-imidazolinon, verwendet werden oder die Reaktion kann in Mischungen von zwei oder mehreren dieser Lösungsmittel ausgeführt werden. Die bevorzugten Lösungsmittel sind Tetrahydrofuran und Diethylether.

Basen, die für diese Reaktion, verwendet werden können, sind z.B. Kaliumcarbonat, Cesiumcarbonat und Natriumhydrid. Die bevorzugte Base ist Natriumhydrid.

Wenigstens ein Äquivalent einer Base (z.B. Kaliumcarbonat, Cesiumcarbonat und Natriumhydrid) im Verhältnis zum Startmaterial der allgemeinen Formel **(VI)** wird verwendet.

Die Reaktion wird normalerweise bei Temperaturen von -78 °C - 150 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einer halben Stunde und 48 Stunden.

Nach Beendigung der Reaktion, werden die Verbindungen **(IV),** von der Reaktionsmischung durch eine der üblichen Trenntechniken getrennt. Falls notwendig werden die Verbindungen durch

Umkristallisation, Destillation oder Chromatographie gereinigt oder können gegebenenfalls auch im nächsten Schritt ohne vorhergehende Reinigung eingesetzt werden.

### Schritt (b)

Eine Möglichkeit das Zwischenprodukt **(III)** aus entsprechenden Verbindungen **(IV)** darzustellen ist in Schema 1 gezeigt.

Eine Verbindung der Formel **(IV)** wird in eine Verbindung der Formel **(III)** durch geeignete Methoden zur Entfernung von Schutzgruppen, die in der Literatur beschrieben sind ("Protective Groups in Organic Synthesis"; Third Edition; Theodora W. Greene, Peter G. M. Wuts; 494-653, und dort zitierte Literatur), überführt.

*t*-Butoxycarbonyl und Benzyloxycarbonyl Schutzgruppen können im sauren Medium entfernt werden (z.B mit Salzsäure oder der Trifluoressigsäure). Acetylschutzgruppen können unter basischen Bedingungen (z.B. mit Kaliumcarbonat oder Cäsiumcarbonat) entfernt werden. Benzylische Schutzgruppen können hydrogenolytisch mit einer Wasserstoffquelle (z.B. Wasserstoff, Ammoniumfomiat, Ameisensäure oder Cyclohexen) in Gegenwart eines Katalysators (z.B. Palladium auf Aktivkohle oder Palladiumhydroxid auf Aktivkohle) entfernt werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester), Amide (z.B. N,N-Dimethylformamid, N,N-Dimethylacetamid), Dimethylsulfoxid, 1,3-Dimethyl-2-imidazolinon, Wasser und Essigsäure verwendet werden oder die Reaktion kann in Mischungen von zwei oder mehreren dieser Lösungsmittel ausgeführt werden.

Säuren, die für diese Reaktion, der Entschützung von t-Butoxycarbonyl und Benzyloxycarbonyl Gruppen verwendet werden können, sind z.B. Trifluoressigsäure, Salzsäure oder andere Säuren, wie sie in der Literatur beschrieben sind (z.B. "Protective Groups in Organic Synthesis"; Third Edition; Theodora W. Greene, Peter G. M. Wuts; S. 494-653).

Die Reaktion wird normalerweise bei Temperaturen von 0 °C - 150 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einer halben Stunde und 72 Stunden. Nach Beendigung der Reaktion, werden die Verbindungen **(III)** von der Reaktionsmischung durch eine der üblichen Trenntechniken getrennt. Falls notwendig werden die Verbindungen durch Umkristallisation, Destillation oder Chromatographie gereinigt oder können, wenn gewünscht, auch im nächsten Schritt ohne vorhergehende Reinigung eingesetzt werden. Es ist außerdem möglich, die Verbindung der allgemeinen Formel **(III)** als Salz zu isolieren, z.B. als Salz der Salzsäure oder der Trifluoressigsäure.

Der gleiche Prozess wird verwendet, um eine Verbindung der Formel **(VIII)** in eine Verbindung der Formel **(VII)** zu überführen.

### Schritt (c)

Eine Möglichkeit, Verbindungen der Formel **(I-a)** aus entsprechenden Verbindungen **(III)** darzustellen, ist in Schema 1 gezeigt.

Eine Verbindung mit der allgemeinen Formel **(I-a),** kann synthetisiert werden, analog zu in der Literatur beschriebenen Vorschriften (siehe z.B. WO 2007/147336), durch eine Kupplungsreaktion einer Verbindung mit der entsprechenden allgemeinen Formel **(III)** mit einem Substrat der allgemeinen Formel **(II)** mit Z = Cl, gegebenenfalls in der Gegenwart eines Säurefängers / Base.

Säurehalogenide **(II)** (Z = Cl) oder die entsprechenden Carbonsäuren **(II)** (Z = OH) sind kommerziell erhältlich oder durch in der Literatur beschriebene Prozesse darstellbar. Außerdem kann ein Substrat mit der allgemeinen Formel **(II),** mit Z = Cl, aus der entsprechenden Säure (Z = OH) durch Chlorierung unter Verwendung literaturbekannter Prozesse dargestellt werden. (z.B. Tetrahedron 2005, 61, 10827-10852, und darin zitierte Literatur).

Als Lösungsmittel können alle üblichen, unter den Reaktionsbedingungen inerten Lösungsmittel, wie z.B. zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol) und Nitrile (z.B. Acetonitril) verwendet werden oder die Reaktion kann in Mischungen von zwei oder mehreren dieser Lösungsmittel ausgeführt werden. Die bevorzugten Lösungsmittel sind Tetrahydrofuran und Dichlormethan.

Wenigstens ein Äquivalent eines Säurefängers / einer Base (z.B. Hünig Base, Triethylamin oder kommerziell erhältliche polymere Säurefänger) im Verhältnis zum Startmaterial der allgemeinen Formel **(III)** wird verwendet. Ist das Startmaterial ein Salz, werden wenigstens zwei Äquivalente des Säurefängers benötigt.

Die Reaktion wird normalerweise bei Temperaturen von 0 °C - 100 °C durchgeführt und vorzugsweise bei 20 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen **(I-a),** von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Alternativ kann eine Verbindung der Formel **(I-a),** auch aus der entsprechenden Verbindung der Formel **(III)** mit einem Substrat der Formel **(II)** mit Z = OH in Gegenwart eines Kupplungsreagenzes synthetisiert werden analog zu in der Literatur beschriebenen Vorschriften (z.B. Tetrahedron 2005, 61, 10827-10852, und darin zitierte Referenzen).

Geeignete Kupplungsreagenzien sind beispielsweise Peptidkupplungsreagenzien (zum Beispiel, N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Brom-tripyrrolidinophosphonium-hexafluorophosphat, O-(7-Azabenzotriazol-1-yl)-N,N,N',N'-tetramethyluroniumhexafluorophosphat, etc.)

Gegebenenfalls kann eine Base, wie z.B. Triethylamin oder Hünig-Base in der Reaktion verwendet werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril) und Amide (z.B. N,N-Dimethylformamid, N,N-Dimethylacetamid) verwendet werden oder die Reaktion kann in Mischungen von zwei oder mehreren dieser Lösungsmittel ausgeführt werden. Das bevorzugte Lösungsmittel ist Dichlormethan.

Die Reaktion wird normalerweise bei Temperaturen von 0 °C - 100 °C durchgeführt und vorzugsweise bei 0 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen **(I-a)** von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Analog lassen sich Verbindungen der Formel **(VII)** in Verbindungen der Formel **(I-c)** überführen.

### Schritt (d)

Eine Möglichkeit Verbindungen der Formel **(I),** in denen Y = S, aus entsprechenden Verbindungen (I), in denen Y = O, darzustellen, ist in Schema 1 gezeigt.

Als Lösungsmittel können alle üblichen, unter den Reaktionsbedingungen inerten Lösungsmittel, wie z.B. Alkohole (z.B. Methanol, Ethanol, Propanol), zyklisch und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester) und Amide (z.B. N,N-Dimethylformamid, N,N-Dimethylacetamid) verwendet werden und die Reaktion kann in Mischungen von zwei oder mehreren dieser Lösungsmittel ausgeführt werden. Die bevorzugten Lösungsmittel sind Chloroform und 1,2-Dimethoxyethan.

Geeignete Schwefelungsreagenzien sind beispielsweise Lawesson's Reagenz (s. z.B. Tetrahedron 1986, 42, 6555-6564) und Phosphorpentasulfid. Das Startmaterial und das Schwefelungsreagenz werden in equimolaren Mengen verwendet, aber das Schwefelungsreagenz kann gegebenenfalls auch im Überschuss verwendet warden.

Die Reaktion wird normalerweise bei Temperaturen von 0 °C - 150 °C durchgeführt und vorzugsweise bei 0 °C - 100 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen **(I)** von der Reaktionsmischung durch eine der üblichen Trenntechniken getrennt. Falls notwendig werden die Verbindungen durch Umkristallisation, Destillation oder Chromatographie gereinigt.

### Schritt (e)

Eine Möglichkeit, Sulfoxiden der Formel (**I-b**) (Y = O) aus entsprechenden Sulfiden **(I-a)** (Y = O) darzustellen, ist in Schema 1 gezeigt.

Die Sulfoxiden (**I-b**) (Y = O) können durch eine Oxidation, aus Sulfiden **(I-a)** (Y = O) mit geeignete Oxidationsmittel hergestellt werden (siehe z.B. WO 1987/005296, J. Med. Chem. 1987, 30(10), 1787-1793).

Als Lösungsmittel können alle üblichen, unter den Reaktionsbedingungen inerten Lösungsmittel, wie z.B. Wasser, Alkohole (z.B. Methanol, Ethanol, Propanol), zyklisch und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), und Amide (z.B. N,NDimethylformamid, N,N-Dimethylacetamid) verwendet werden und die Reaktion kann in Mischungen von zwei oder mehreren dieser Lösungsmittel ausgeführt werden. Die bevorzugten Lösungsmittel sind Tetrahydrofuran, Dichlormethan und Ethanol.

Geeignete Oxidationsmittel sind beispielsweise Natrium(meta)periodat und 3-Chlor-perbenzosäure. Das Startmaterial und das Oxidationsmittel werden in equimolaren Mengen verwendet, aber das Natrium(meta)periodat kann gegebenenfalls auch im Überschuss verwendet warden.

Die Reaktion wird normalerweise bei Temperaturen von 0 °C - 100 °C durchgeführt und vorzugsweise bei 20 °C - 40 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**I-b**) (Y = O), von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Analog lassen sich Sulfide der Formel **(I-a)** (Y = S) in Sulfoxide der Formel (**I-b**) (Y = S) überführen.

### Schritt (f)

Eine Möglichkeit, Sulfonen der Formel **(I-c)** (Q = A-G-C(=Y)-), aus entsprechenden Sulfiden **(I-a)** (Q = A-G-C(=Y)-), darzustellen ist in Schema 1 gezeigt.

Die Sulfone **(I-c)** (Q = A-G-C(=Y)-), können durch eine Oxidation aus den Sulfiden der Formel **(I-a)** (Q = A-G-C(=Y)-), mit geeignete Oxidationsmittel hergestellt werden (siehe z.B. Tetrahedron 2006, 62(50),11592-11598).

Als Lösungsmittel können alle üblichen, unter den Reaktionsbedingungen inerten Lösungsmittel, wie z.B. Wasser, Alkohole (z.B. Methanol, Ethanol, Propanol), zyklisch und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), und Amide (z.B. N,NDimethylformamid, N,N-Dimethylacetamid) verwendet werden und die Reaktion kann in Mischungen von zwei oder mehreren dieser Lösungsmittel ausgeführt werden. Die bevorzugten Lösungsmittel sind Tetrahydrofuran, Dichlormethan und Ethanol.

Geeignete Oxidationsmittel sind beispielsweise Wasserstoffperoxid mit Ammoniummolybdat in Ethanol und 3-Chlor-perbenzosäure. Das Startmaterial und das Oxidationsmittel werden in wenigstens zwei Äquivalent Mengen verwendet, aber die Oxidationsmittel können gegebenenfalls auch im Überschuss verwendet werden.

Die Reaktion wird normalerweise bei Temperaturen von 0 °C - 100 °C durchgeführt und vorzugsweise bei 20 °C - 40 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Sulfone **(I-c)** (Q = A-G-C(=Y)-), von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Analog lassen sich Sulfide der Formel **(III)** oder **(IV),** in Sulfone der Formel **(VII)** oder **(VIII)** überführen.

### Schritt (g)

Der gleiche Prozess kann verwendet werden, um ein Sulfoxid der Formel **(I-b),** in eine Sulfon der allgemeinen Formel **(I-c),** zu überführen (Schema 1).

Die erfindungsgemäßen Verfahren zur Herstellung der Verbindungen der Formel **(I)** werden vorzugsweise unter Verwendung eines oder mehrerer Reaktionshilfsmittel durchgeführt.

Als Reaktionshilfsmittel kommen gegebenenfalls die üblichen anorganischen oder organischen Basen oder Säureakzeptoren in Betracht. Hierzu gehören vorzugsweise Alkalimetall- oder Erdalkalimetall-acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calciumamid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-düsopropylamin, N,N-Dimethylcyclohexylamin, Dicyclohexylamin, Ethyl-dicyclohexylamin, N,N-Dimethyl-anilin, N,N-Dimethylbenzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Die erfindungsgemäßen Verfahren werden vorzugsweise unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl- oder Methyl-isobutyl-keton, Ester wie Essigsäuremethylester oder -ethylester, Nitrile wie z.B. Acetonitril oder Propionitril, Amide wie z.B. Dimethylformamid, Dimethylacetamid und N-Methylpyrrolidon, sowie Dimethylsulfoxid, Tetramethylensulfon und Hexamethylphosphorsäuretriamid und DMPU.

Die Reaktionstemperaturen können bei den erfindungsgemäßen Verfahren in einem größeren Bereich variiert werden. Im Allgemeinen arbeitet man bei Temperaturen zwischen 0°C und 250°C, vorzugsweise bei Temperaturen zwischen 10°C und 185°C.

Die erfindungsgemäßen Verfahren werden im allgemeinen unter Normaldruck durchgeführt. Es ist jedoch auch möglich, unter erhöhtem oder vermindertem Druck zu arbeiten.

Zur Durchführung der erfindungsgemäßen Verfahren werden die jeweils benötigten Ausgangsstoffe im Allgemeinen in angenähert äquimolaren Mengen eingesetzt. Es ist jedoch auch möglich, eine der jeweils eingesetzten Komponenten in einem größeren Überschuss zu verwenden. Die Aufarbeitung erfolgt bei den erfindungsgemäßen Verfahren jeweils nach üblichen Methoden (vgl. die Herstellungsbeispiele).

Ein weiterer Gegenstand der Erfindung betrifft die nichtmedizinische Verwendung der erfindungsgemäßen Thiazolylpiperidin Derivate zum Bekämpfen unerwünschter Mikroorganismen.

Ein weiterer Gegenstand der Erfindung betrifft ein Mittel zum Bekämpfen unerwünschter Mikroorganismen, umfassend wenigstens ein Thiazolylpiperidin Derivate gemäß der vorliegenden Erfindung.

Außerdem betrifft die Erfindung ein Verfahren zum Bekämpfen unerwünschter Mikroorganismen, **dadurch gekennzeichnet, dass** die erfindungsgemäßen Thiazolylpiperidin Derivate auf die Mikroorganismen und/oder in deren Lebensraum ausgebracht werden.

Weiterhin betrifft die Erfindung ein Saatgut, welches mit wenigstens einem erfindungsgemäßen Thiazolylpiperidin Derivate behandelt wurde.

Ein letzter Gegenstand der Erfindung betrifft ein Verfahren zum Schutz von Saatgut vor unerwünschten Mikroorganismen durch Verwendung eines mit wenigstens einem Thiazolylpiperidin Derivate gemäß der vorliegenden Erfindung behandelten Saatgutes.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Die erfindungsgemäßen Thiazolylpiperidin Derivate der Formel (**I**) besitzen sehr gute fungizide Eigenschaften und lassen sich im Pflanzenschutz beispielsweise zur Bekämpfung von Plasmodiophoromyceten, Oomyceten, Chytridiomyceten, Zygomyceten, Ascomyceten, Basidiomyceten und Deuteromyceten einsetzen.

Bakterizide lassen sich im Pflanzenschutz beispielsweise zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen..

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi oder Phakopsora meibomiae; Puccinia-Arten, wie beispielsweise Puccinia recondita, Puccinia graminis oder Puccinia striiformis; Uromyces-Arten, wie beispielsweise Uromyces appendiculatus;
Erkrankungen, hervorgerufen durch Erreger der Gruppe der Oomyceten wie z.B. Albugo-Arten, wie beispielsweise Albugo candida; Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladosporium-Arten, wie beispielsweise Cladosporium cucumerinum; Cochliobolus-Arten, wie beispielsweise Cochliobolus sativus (Konidienform: Drechslera, Syn: Helminthosporium) oder Cochliobolus miyabeanus; Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola, Mycosphaerella arachidicola oder Mycosphaerella fijiensis; Phaeosphaeria-Arten, wie beispielsweise Phaeosphaeria nodorum; Pyrenophora-Arten, wie beispielsweise Pyrenophora teres oder Pyrenophora tritici repentis; Ramularia-Arten, wie beispielsweise Ramularia collo-cygni oder Ramularia areola; Rhynchosporium-Arten, wie beispielsweise Rhynchosporium secalis; Septoria-Arten, wie beispielsweise Septoria apii oder Septoria lycopersici; Stagonospora-Arten, wie beispielsweise Stagonospora nodorum; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Plasmodiophora-Arten, wie beispielsweise Plasmodiophora brassicae; Rhizoctonia-Arten, wie beispielsweise Rhizoctonia solani; Sarocladium-Arten, wie beispielsweise Sarocladium oryzae; Sclerotium-Arten, wie beispielsweise Sclerotium oryzae; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Stagonospora-Arten, wie beispielsweise Stagonospora nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries oder Tilletia controversa; Urocystis-Arten, wie beispielsweise Urocystis occulta; Ustilago-Arten, wie beispielsweise Ustilago nuda;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum oder Penicillium purpurogenum; Rhizopus -Arten, wie beispielsweise Rhizopus stolonifer; Sclerotinia-Arten, wie beispielsweise Sclerotinia sclerotiorum; Verticilium-Arten, wie beispielsweise Verticilium alboatrum;
Samen- und bodenbürtige Fäulen und Welken, sowie Sämlingserkrankungen, hervorgerufen durch z.B. Alternaria-Arten, wie beispielsweise Alternaria brassicicola; Aphanomyces-Arten, wie beispielsweise Aphanomyces euteiches; Ascochyta-Arten, wie beispielsweise Ascochyta lentis; Aspergillus-Arten, wie beispielsweise Aspergillus flavus; Cladosporium-Arten, wie beispielsweise Cladosporium herbarum; Cochliobolus-Arten, wie beispielsweise Cochliobolus sativus (Konidienform: Drechslera, Bipolaris Syn: Helminthosporium); Colletotrichum-Arten, wie beispielsweise Colletotrichum coccodes; Fusarium-Arten, wie beispielsweise Fusarium culmorum; Gibberella-Arten, wie beispielsweise Gibberella zeae; Macrophomina-Arten, wie beispielsweise Macrophomina phaseolina; Microdochium-Arten, wie beispielsweise Microdochium nivale; Monographella-Arten, wie beispielsweise Monographella nivalis; Penicillium-Arten, wie beispielsweise Penicillium expansum; Phoma-Arten, wie beispielsweise Phoma lingam; Phomopsis-Arten, wie beispielsweise Phomopsis sojae; Phytophthora Arten, wie beispielsweise Phytophthora cactorum; Pyrenophora-Arten, wie beispielsweise Pyrenophora graminea; Pyricularia-Arten, wie beispielsweise Pyricularia oryzae; Pythium-Arten, wie beispielsweise Pythium ultimum; Rhizoctonia-Arten, wie beispielsweise Rhizoctonia solani; Rhizopus-Arten, wie beispielsweise Rhizopus oryzae; Sclerotium-Arten, wie beispielsweise Sclerotium rolfsii; Septoria-Arten, wie beispielsweise Septoria nodorum; Typhula-Arten, wie beispielsweise Typhula incarnata; Verticillium-Arten, wie beispielsweise Verticillium dahliae;
Krebserkrankungen, Gallen und Hexenbesen, hervorgerufen durch z.B. Nectria-Arten, wie beispielsweise Nectria galligena;

Welkeerkrankungen hervorgerufen durch z.B. Monilinia-Arten, wie beispielsweise Monilinia laxa;

Deformationen von Blättern, Blüten und Früchten, hervorgerufen durch z.B. Exobasidium-Arten, wie beispielsweise Exobasidium vexans; Taphrina-Arten, wie beispielsweise Taphrina deformans;

Degenerationserkrankungen holziger pflanzen, hervorgerufen durch z.B. Esca-Arten, wie beispielsweise Phaeomoniella chlamydospora, Phaeoacremonium aleophilum oder Fomitiporia mediterranea; Ganoderma-Arten, wie beispielsweise Ganoderma boninense;

Blüten- und Samenerkrankungen, hervorgerufen durch z.B. Botrytis-Arten, wie beispielsweise Botrytis cinerea;

Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;

Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;

Bevorzugt können die folgenden Krankheiten von Soja-Bohnen bekämpft werden:
Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze und Bakterien zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst-, Kartoffel- und Gemüseanbau, wie beispielsweise insbesondere gegen falsche Mehltaupilze, Oomyceten, wie beispielsweise Phytophthora-, Plasmopara-, Pseudoperonospora- und Pythium-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Insektizide verwenden. Sie lassen sich gegebenenfalls auch als Zwischen- oder Vorprodukte für die Synthese weiterer Wirkstoffe einsetzen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes in der Landwirtschaft, im Gartenbau, bei der Tierzucht, in Forsten, in Gärten und Freizeiteinrichtungen, im Vorrats- und Materialschutz sowie auf dem Hygienesektor. Sie können vorzugsweise als Pflanzenschutzmittel eingesetzt werden. Sie sind gegen normal sensible und resistente Arten sowie gegen alle oder einzelne Entwicklungsstadien wirksam.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Das erfindungsgemäße Verfahren zum Bekämpfen von unerwünschten Pilzen kann auch zum Schutz von so genannte Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die vorliegende Erfindung betrifft weiterhin ein Mittel zum Bekämpfen unerwünschter Mikroorganismen, umfassend wenigstens eines der erfindungsgemäßen Thiazolylpiperidin Derivate. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Die Wirkstoffe können als solche, in Form ihrer Formulierungen oder den daraus bereiteten Anwendungsformen, wie gebrauchsfertige Lösungen, Emulsionen, wasser- oder ölbasierte Suspensionen, Pulver, Spritzpulver, Pasten, lösliche Pulver, Stäubemittel, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die zuvor beschriebenen Formulierungen können in einem erfindungsgemäßen Verfahren zum Bekämpfen unerwünschter Mikroorganismen verwendet werden, bei dem die erfindungsgemäßen Thiazolylpiperidin Derivate auf die Mikroorganismen und/oder in deren Lebensraum ausgebracht werden.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Als Mischpartner kommen zum Beispiel bekannte Fungizide, Insektizide, Akarizide, Nematizide oder auch Bakterizide (siehe auch Pesticide Manual, 14th ed.) infrage.

Auch eine Mischung mit anderen bekannten Wirkstoffen, wie Herbiziden, oder mit Düngemitteln und Wachstumsregulatoren, Safenern bzw. Semiochemicals ist möglich.

Die Anwendung geschieht in einer den Anwendungsformen angepaßten üblichen Weise.

Die Erfindung umfasst weiterhin ein Verfahren zur Behandlung von Saatgut.

Ein weiterer Aspekt der vorliegenden Erfindung betrifft insbesondere ein Saatgut, welches mit wenigstens einem der erfindungsgemäßen Thiazolylpiperidin Derivate behandelt ist. Die erfindungsgemäßen Saatgüter finden Anwendung in Verfahren zum Schutz von Saatgut vor pflanzenpathogenen Schadpilzen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von pflanzenpathogenen Schadpilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von tierischen Schädlingen und/oder pflanzenpathogenen Schadpilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von tierischen Schädlingen und/oder pflanzenpathogenen Schadpilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor tierischen Schädlingen und/oder pflanzenpathogenen Schadpilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

Darüber hinaus weisen die erfindungsgemäßen Verbindungen der Formel (**I**) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe der Formel (**I**) können daher sowohl in medizinischen als auch in nicht-medizinischen Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiterhin können die erfindungsgemäßen Verbindungen allein oder in Kombinationen mit anderen Wirkstoffen als Antifouling-Mittel eingesetzt werden.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden. Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosateacetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei:
   http://www.lifesci.sussex.ac.uk/Home/Neil Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil-Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfojrc.it/gmp_browse.aspx und http://www.agbios.com/dbase.php).

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Die Herstellung und die Verwendung der erfindungsgemäßen Wirkstoffe der Formeln (I) geht aus den folgenden Beispielen hervor. Die Erfindung ist jedoch nicht auf diese Beispiele beschränkt.

Allgemeines: Wenn nicht anders angegeben, werden alle chromatografischen Reinigungs- bzw. Trennungsschritte an Kieselgel und mit einem Lösungsmittelgradienten von 0: 100 Essigsäure-ethylester/Cyclohexan zu 100:0 Essigsäure-ethylester/Cyclohexan durchgeführt.

**Herstellung von Ausgangsstoffen der Formel (VI):**

### tert-Butyl-4-[4-(iodmethyl)-1,3-thiazol-2-yl]piperidin-1-carboxylat (VI-1)

Zu einer Lösung von Triphenylphospin (193 mg) und Imidazol (54.7 mg) in Dichlormethan (5 mL) wird Iod (187 mg) bei Raumtemperatur gegeben. Die Reaktionsmischung wird bei dieser Temperatur gerührt, bis das Iod völlig gelöst ist. tert-Butyl-4-[4-(hydroxymethyl)-1,3-thiazol-2-yl]piperidin-1-carboxylat (200 mg) wird in einer Portion zugegeben und für 30 Minuten gerührt, bevor das Lösungsmittel unter reduziertem Druck entfernt wird. Nach säulenchromatographischer Reinigung erhält man tert-Butyl-4-[4-(iodmethyl)-1,3-thiazol-2-yl]piperidin-1-carboxylat (199 mg, 74%).
logP (pH2.7): 3.71
¹H NMR (DMSO-d₆, 400 MHz): δₚₚₘ : 1.41 (s, 9H), 1.55 (qd, 2H), 2.01 (dd, 2H), 2.91 (m, 2H), 3.16 (m, 1H), 3.93-4.02 (m, 2H), 4.58 (s, 2H), 7.53 (s, 1H)
MS (ESI): 353 ([M+H]⁺)

### Herstellung von Ausgangsstoffen der Formel (IV):

### tert-Butyl-4-{4-[(1-naphthylsulfanyl)methyl]-1,3-thiazol-2-yl}piperidin-1-carboxylat (IV-1)

Zu einer Lösung von Naphthylthiol (168 mg) in Tetrahydrofuran (3 mL) wird bei Raumtemperatur unter Argonatmosphäre Natriumhydrid (39.8 mg) gegeben. Nach Beendigung der Gasentwicklung wird eine Lösung von tert-Butyl-4-[4-(iodmethyl)-1,3-thiazol-2-yl]piperidin-1-carboxylat (356 mg) in Tetrahydrofuran langsam zugetropft. Das Reaktionsgemisch wird über 30 Minuten bei Raumtemperatur gerührt, dann 3 g Kieselgel zugegeben und das Lösungsmittel im Vakuum entfernt. Der Rückstand wird mittels Säulenchromatographie gereinigt. Man erhält tert-Butyl-4-{4-[(1-naphthylsulfanyl)methyl]-1,3-thiazol-2-yl}piperidin-1-carboxylat (410 mg, 100%).
logP (pH2.7): 5.33

¹H NMR (CD₃CN, 400 MHz): δₚₚₘ : 1.44 (s, 9H), 1.55 (qd, 2H), 1.95-2.05 (m, 2H), 2.88 (td, 2H), 3.08 (m, 1H), 3.99-4.06 (m, 2H), 4.23 (s, 2H), 6.85 (s, 1H), 7.40 (dd, 1H), 7.49-7.55 (m, 2H), 7.62 (dd, 1H), 7.79 (d, 1H), 7.87 (m, 1H), 8.33 (m, 1H)
MS (ESI): 441 ([M+H]⁺)
**tert-Butyl-4-(4-{[(3S,5S,7S)-adamantan-1-ylsulfanyl]methyl}-1,3-thiazol-2-yl)piperidin-1-carboxylat (IV-2)**
logP (pH2.7): 6.66
¹H NMR (CD₃CN, 400 MHz): δₚₚₘ : 1.43 (s, 9H), 1.55-1.68 (m, 2H), 1.68-21.75 (m, 6H), 1.88-1.90 (m, 6H), 2.05 (bs, 3H), 2.90 (td, 2H), 3.12 (m, 1H), 3.83 (s, 2H), 4.07 (dt, 2H), 7.09 (s, 1H)
MS (ESI): 449 ([M+H]⁺)
**tert-Butyl-4-{4-[(cyclohexylsulfanyl)methyl]-1,3-thiazol-2-yl}piperidin-1-carboxylat (IV-3)**
logP (pH2.7): 5.45
¹H NMR (CD₃CN, 400 MHz): δₚₚₘ : 1.23-1.35 (m, 6H), 1.43 (s, 9H), 1.55-1.78 (m, 6H), 2.72 (m, 1H), 2.91 (td, 2H), 3.14 (m, 1H), 3.81 (s, 2H), 4.07 (dt, 2H), 7.08 (s, 1H)
MS (ESI): 397 ([M+H]⁺)
**tert-Butyl-4-(4-{[(1-phenyl-1H-tetrazol-5-yl)sulfanyl]methyl}-1,3-thiazol-2-yl)piperidin-1-carboxylat (IV-4)**
logP (pH2.7): 3.91
¹H NMR (CD₃CN, 400 MHz): δₚₚₘ : 1.43 (s, 9H), 1.57 (qd, 2H), 1.92-2.05 (m, 2H), 2.88 (td, 2H), 3.10 (m, 1H), 4.05 (dt, 2H), 4.59 (s, 2H), 7.24 (s, 1H), 7.51-7.55 (m, 2H), 7.58-7.61 (m, 3H)
MS (ESI): 459 ([M+H]⁺)
**tert-Butyl-4-{4-[(chinolin-8-ylsulfanyl)methyl]-1,3-thiazol-2-yl}piperidin-1-carboxylat (IV-5)**
logP (pH2.7): 3.71
¹H NMR (CD₃CN, 400 MHz): δₚₚₘ : 1.44 (s, 9H), 1.62 (qd, 2H), 1.98-2.05 (m, 2H), 2.90 (td, 2H), 3.14 (m, 1H), 4.06 (dt, 2H), 4.39 (s, 2H), 7.17 (s, 1H), 7.46-7.51 (m, 2H), 7.65-7.70 (m, 2H), 8.22 (dd, 1H), 8.87 (dd, 1H)
MS (ESI): 442 ([M+H]⁺)

### Herstellung von Verbindungen der Formel (I):

### 2-[5-Methyl-3-(trifluormethyl)-1H-pyrazol-1-yl]-1-(4-{4-[(1-naphthylsulfanyl)methyl]-1,3-thiazol-2-yl}piperidin-1-yl)ethanon (I-1)

Zu tert-Butyl-4-{4-[(1-naphthylsulfanyl)methyl]-1,3-thiazol-2-yl}piperidin-1-carboxylat (269 mg), wird tropfenweise bei Raumtemperatur eine Lösung von Trifluoressigsäure-Lösung (30% in Dichlormethan, 2 mL) zugegeben. Die Reaktionsmischung wird 30 Minuten lang gerührt, dann wird Triethylamin (2 mL) dem Reaktionsgemisch zugegeben.

Zu einer Lösung von [5-Methyl-3-(trifluormethyl)-1H-pyrazol-1-yl]essigsäure (140 mg) in Dichlormethan (5 mL) wird Oxalylchlorid (232 mg) und ein Tropfen N,N-Dimethylformamid gegeben. Dann wird das Reaktionsgemisch für 30 Minuten gerührt. Dann wird der Überschuss Oxalylchlorid unter vermindertem Druck entfernt und der Rückstand wird wieder in Dichlormethan (1 mL) gelöst. Die Lösung wird dann zu der ersterer Lösung aus Trifluoressigsäure-4-{4-[(1-naphthylsulfanyl)methyl]-1,3-thiazol-2-yl}piperidin(1:1) (**III-1**) in Dichlormethan und Triethylamin gegeben. Die Reaktionsmischung lässt man dann auf Raumtemperatur erwärmen und rührt weitere 20 Stunden. Dann wird das Lösungsmittel unter vermindertem Druck entfernt. Der Rückstand wird säulenchromatographisch gereinigt. Man erhält 2-[5-Methyl-3-(trifluormethyl)-1H-pyrazol-1-yl]-1-(4-{4-[(1-naphthylsulfanyl)methyl]-1,3-thiazol-2-yl}piperidin-1-yl)ethanon (134 mg, 36%).
logP (pH2.7): 4.33
1H NMR (CD3CN, 400 MHz): δₚₚₘ : 1.50-1.80 (m, 2H), 2.15-2.25 (m, 2H), 2.23 (s, 3H), 2.90 (bs, 1H), 3.20 (m, 1H), 3.25 (bs, 1H), 3.90 (bs, 1H), 4.25 (s, 2H), 4.38 (bs, 1H), 5.04 (bs, 2H), 6.37 (s, 1H), 6.87 (s, 1H), 7.41 (dd, 1H), 7.48-7.56 (m, 2H), 7.63 (d, 1H), 7.80 (d, 1H), 7.89 (m, 1H), 8.33 (s, 1H)
MS (ESI): 531 ([M+H]⁺)

### 1-[4-(4-{[(3S,5S,7S)-Adamantan-1-ylsulfanyl]methyl}-1,3-thiazol-2-yl)piperidin-1-yl]-2-[5-methyl-3-(trifluormethyl)-1H-pyrazol-1-yl]ethanon (I-2)

logP (pH2.7): 5.21

¹H NMR (CD₃CN, 400 MHz): δₚₚₘ : 1.60-1.85 (m, 14H), 2.00-2.05 (m, 3H), 2.05-2.18 (m, 2H), 2.23 (s, 3H), 2.90 (bs, 1H), 3.25 (m, 2H), 3.64 (s, 2H), 3.92 (bs, 1H), 4.42 (bs, 1H), 5.03 (bs, 2H), 6.36 (s, 1H), 7.11 (s, 1H) MS (ESI): 539 ([M+H]⁺)

### 1-(4-{4-[(Cyclohexylsulfanyl)methyl]-1,3-thiazol-2-yl}piperidin-1-yl)-2-[5-methyl-3-(trifluormethyl)-1H-pyrazol-1-yl]ethanon (I-3)

logP (pH2.7): 4.30

¹H NMR (CD₃CN, 400 MHz): δ_{ppm :} 1.24-1.38 (m, 6H), 1.54-1.88 (m, 6H), 2.05-2.18 (m, 2H), 2.23 (s, 3H), 2.73 (m, 1H), 2.90 (bs, 1H), 3.26 (m, 1H), 3.28 (bs, 1H), 3.82 (s, 2H), 3.92 (bs, 1H), 4.41 (bs, 1H), 5.03 (bs, 2H), 6.36 (s, 1H), 7.10 (s, 1H)
MS (ESI): 487 ([M+H]⁺)

### 2-[5-Methyl-3-(trifluormethyl)-1H-pyrazol-1-yl]-1-[4-(4-{[(1-phenyl-1H-tetrazol-5-yl)sulfanyl]methyl}-1,3-thiazol-2-yl)piperidin-1-yl]ethanon (I-4)

logP (pH2.7): 3.25
1H NMR (CD3CN, 400 MHz): δₚₚₘ : 1.55-1.86 (m, 2H), 2.05-2.13 (m, 2H), 2.23 (s, 3H), 2.89 (bs, 1H), 3.23 (m, 1H), 3.25 (bs, 1H), 3.90 (bs, 1H), 4.38 (bs, 1H), 4.60 (s, 2H), 5.02 (bs, 2H), 6.36 (s, 1H), 7.26 (s, 1H), 7.51-7.57 (m, 2H), 7.58-7.62 (m, 3H)
MS (ESI): 549 ([M+H]+)

### 1-(4-{4-[(Chinolin-8-ylsulfanyl)methyl]-1,3-thiazol-2-yl}piperidin-1-yl)-2-[5-methyl-3-(trifluormethyl)-1H-pyrazol-1-yl]ethanon (I-5)

logP (pH2.7): 3.00

¹H NMR (CD₃CN, 400 MHz): δₚₚₘ : 1.60-1.90 (m, 2H), 2.05-2.15 (m, 2H), 2.23 (s, 3H), 2.90 (bs, 1H), 3.20-3.30 (m, 2H), 3.92 (bs, 1H), 4.40 (bs, 3H), 5.03 (bs, 2H), 6.36 (s, 1H), 7.19 (s, 1H), 7.45-7.51 (m, 2H), 7.65-7.69 (m, 2H), 8.23 (dd, 1H), 8.86 (dd, 1H)

MS (ESI): 532 ([M+H]⁺)

### 2-[5-Methyl-3-(trifluormethyl)-1H-pyrazol-1-yl]-1-(4-{4-[(1-naphthylsulfonyl)methyl]-1,3-thiazol-2-yl}piperidin-1-yl)ethanon (I-10)

Zu einer Lösung von 2-[5-Methyl-3-(trifluormethyl)-1H-pyrazol-1-yl]-1-(4-{4-[(1-naphthylsulfanyl)-methyl]-1,3-thiazol-2-yl}piperidin-1-yl)ethanon (160 mg) in Ethanol (2 ml), werden bei Raumtemperatur Ammoniummolybdat (50.4 mg) und Wasserstoffperoxid (0.26 mL) gegeben. Die Reaktionsmischung wird 24 Stunden gerührt. Dann wird gesättigte wässrige Natriumchlorid-Lösung (5 mL) zu dem Reaktionsgemisch gegeben. Die wässrige Phase wird abgetrennt und mit Dichlormethan extrahiert. Die gesamten organischen Phasen werden vereinigt und mit wasserfreiem Natriumsulfat getrocknet. Anschließend wird der Feststoff abfiltriert und das Lösungsmittel unter reduziertem Druck entfernt. Nach säulenchromatographischer Reinigung erhält man 2-[5-Methyl-3-(trifluormethyl)-1H-pyrazol-1-yl]-1-(4-{4-[(1-naphthylsulfonyl)methyl]-1,3-thiazol-2-yl}piperidin-1-yl)ethanon (99.6 mg, 57%).
logP (pH2.7): 3.21
1H NMR (CD3CN, 400 MHz): δₚₚₘ: 1.20-1.46 (m, 2H) 1.70-1.85 (m, 2H), 2.24 (s, 3H), 2.78 (bs, 1H), 2.95 (m, 1H), 3.12 (bs, 1H), 3.69 (bs, 1H), 4.15 (bs, 1H), 4.72 (s, 2H), 4.99 (bs, 2H), 6.38 (s, 1H), 7.22 (s, 1H), 7.55 (t, 1H), 7.61-7.68 (m, 2H), 8.00-8.05 (m, 2H), 8.19 (d, 1H), 8.66 (d, 1H)
MS (ESI): 563 ([M+H]+)

### 1-[4-(4-{[(3S,5S,7S)-Adamantan-1-ylsulfonyl]methyl}-1,3-thiazol-2-yl)piperidin-1-yl]-2-[5-methyl-3-(trifluormethyl)-1H-pyrazol-1-yl]ethanon (I-6)

logP (pH2.7): 3.42
1H NMR (DMSO-d6, 400 MHz): δₚₚₘ : 1.50-1.90 (m, 8H) 1.95-1.97 (m, 6H), 2.06-2.12 (m, 5H), 2.22 (s, 3H), 2.90 (bs, 1H), 3.28 (bs, 1H), 3.34 (m, 1H), 3.98 (bs, 1H), 4.30 (bs, 1H), 4.48 (s, 2H), 5.21 (bs, 2H), 6.44 (s, 1H), 7.55 (s, 1H)
MS (ESI): 571 ([M+H]+) **1-(4-{4-[(Cyclohexylsulfonyl)methyl]-1,3-thiazol-2-yl}piperidin-1-yl)-2-[5-methyl-3-(trifluormethyl)-1H-pyrazol-1-yl]ethanon (I-7)**
logP (pH2.7): 2.90
¹H NMR (CD₃CN, 400 MHz): δₚₚₘ : 1.18-1.47 (m, 3H), 1.42-1.52 (m, 2H), 1.65-1.95 (m, 3H), 2.10-2.19 (m, 4H), 2.23 (s, 3H), 2.92 (bs, 1H), 3.00 (tt, 1H), 3.30 (bs, 1H), 3.31 (tt, 1H), 3.92 (bs, 1H), 4.36 (s, 2H), 4.40 (bs, 1H), 5.04 (bs, 2H), 6.36 (s, 1H), 7.39 (s, 1H)
MS (ESI): 519 ([M+H]⁺)

### 2-[5-Methyl-3-(trifluormethyl)-1H-pyrazol-1-yl]-1-[4-(4-{[(1-phenyl-1H-tetrazol-5-yl)sulfonyl]methyl}-1,3-thiazol-2-yl)piperidin-1-yl]ethanon (I-8)

logP (pH2.7): 3.10
¹H NMR (CD₃CN, 400 MHz): δₚₚₘ : 1.48-1.70 (m, 2H), 1.90-2.10 (m, 2H), 2.24 (s, 3H), 2.84 (bs, 1H), 3.18 (m, 1H), 3.22 (bs, 1H), 3.88 (bs, 1H), 4.37 (bs, 1H), 4.87 (s, 2H), 5.03 (bs, 2H), 6.36 (s, 1H), 7.44 (s, 1H), 7.45-7.52 (m, 2H), 7.55-7.68 (m, 3H)
MS (ESI): 581 ([M+H]⁺)

### 1-(4-{4-[(Chinolin-8-ylsulfonyl)methyl]-1,3-thiazol-2-yl}piperidin-1-yl)-2-[5-methyl-3-(trifluormethyl)-1H-pyrazol-1-yl]ethanon (I-9)

logP (pH2.7): 2.63
¹H NMR (CD₃CN, 400 MHz): δₚₚₘ: 1.18 (qd, 1H), 1.33 (qd, 1H), 1.68 (bd, 1H), 1.77 (bd, 1H), 2.23 (s, 3H), 2.76 (td, 1H), 2.99 (m, 1H), 3.10 (m, 1H), 3.60 (bd, 1H), 4.08 (bd, 1H), 4.98 (d, 1H), 5.04 (d, 1H), 5.27 (s, 2H), 6.41 (s, 1H), 7.20 (s, 1H), 7.65 (dd, 1H), 7.68 (dd, 1H), 8.17 (dd, 1H), 8.25 (dd, 1H), 8.46 (dd, 1H), 9.14 (dd, 1H)
MS (ESI): 564 ([M+H]⁺)

### 2-[5-Methyl-3-(trifluormethyl)-1H-pyrazol-1-yl]-1-(4-{4-[(1-naphthylsulfinyl)methyl]-1,3-thiazol-2-yl}piperidin-1-yl)ethanon (I-13)

Zu einer Lösung von 2-[5-Methyl-3-(trifluormethyl)-1H-pyrazol-1-yl]-1-(4-{4-[(1-naphthylsulfanyl)-methyl]-1,3-thiazol-2-yl}piperidin-1-yl)ethanon (105 mg) in Tetrahydrofuran (1 mL) und Methanol (1 ml), wird bei Raumtemperatur eine Lösung von Natriummetaperiodat (50.6 mg) in Wasser (1 mL) gegeben. Die Reaktionsmischung wird 20 Stunden gerührt. Dann wird gesättigte wässrige Natriumchlorid-Lösung (5 mL) zu dem Reaktionsgemisch gegeben. Die wässrige Phase wird abgetrennt und mit Dichlormethan extrahiert. Die gesamten organischen Phasen werden vereinigt und mit wasserfreiem Natriumsulfat getrocknet. Anschließend wird der Feststoff abfiltriert und das Lösungsmittel unter reduziertem Druck entfernt. Nach säulenchromatographischer Reinigung erhält man 2-[5-Methyl-3-(trifluormethyl)-1H-pyrazol-1-yl]-1-(4-{4-[(1-naphthylsulfinyl)methyl]-1,3-thiazol-2-yl}piperidin-1-yl)ethanon (93 mg, 82%).
logP (pH2.7): 2.86

1H NMR (CD3CN, 400 MHz): δₚₚₘ : 1.30-1.60 (m, 2H) 1.82-1.98 (m, 2H), 2.24 (s, 3H), 2.83 (bs, 1H), 3.05 (m, 1H), 3.18 (bs, 1H), 3.80 (bs, 1H), 4.25 (bs, 1H), 4.29 (d, 1H), 4.39 (d, 1H), 5.02 (bs, 2H), 6.38 (s, 1H), 7.01 (s, 1H), 7.52-7.61 (m, 3H), 7.81 (dd, 1H), 7.92 (d, 1H), 7.98 (d, 1H), 8.01 (d, 1H)
MS (ESI): 547 ([M+H]+)

### 2-[5-Methyl-3-(trifluormethyl)-1H-pyrazol-1-yl]-1-[4-(4-{[(1-phenyl-1H-tetrazol-5-yl)sulfinyl]methyl}-1,3-thiazol-2-yl)piperidin-1-yl]ethanon (I-14)

logP (pH2.7): 2.69
1H NMR (CD3CN, 400 MHz): δₚₚₘ: 1.50 (m, 1H), 1.64 (qd, 1H), 1.95-2.05 (m, 2H), 2.22 (s, 3H), 2.81 (td, 1H), 3.15 (tt, 1H), 3.22 (td, 1H), 3.87 (bd, 1H), 4.31 (bd, 1H), 4.70 (s, 2H), 5.02 (d, 1H), 5.09 (d, 1H), 6.39 (s, 1H), 7.33 (s, 1H), 7.50-7.54 (m, 2H), 7.59-7.66 (m, 3H)

### MS (ESI): 565 ([M+H]+)

### 1-(4-{4-[(Cyclohexylsulfinyl)methyl]-1,3-thiazol-2-yl}piperidin-1-yl)-2-[5-methyl-3-(trifluormethyl)-1H-pyrazol-1-yl]ethanon (I-11)

logP (pH2.7): 2.55
1H NMR (CD3CN, 400 MHz): δₚₚₘ: 1.21-1.50 (m, 4H), 1.63-1.90 (m, 8H), 2.10-2.19 (m, 2H), 2.23 (s, 3H), 2.58 (tt, 1H), 2.92 (bs, 1H), 3.29 (tt, 1H), 3.35 (bs, 1H), 3.95 (bs, 1H), 3.98 (d, 1H), 4.09 (d, 1H), 4.42 (bs, 1H), 5.04 (bs, 2H), 6.36 (s, 1H), 7.25 (s, 1H)

### MS (ESI): 503 ([M+H]⁺)

### 1-(4-{4-[(Chinolin-8-ylsulfinyl)methyl]-1,3-thiazol-2-yl}piperidin-1-yl)-2-[5-methyl-3-(trifluormethyl)-1H-pyrazol-1-yl]ethanon (I-12)

logP (pH2.7): 2.48
¹H NMR (CD₃CN, 400 MHz): δₚₚₘ : 1.35-1.54 (m, 2H), 1.75-2.05 (m, 2H), 2.24 (s, 3H), 2.82 (bs, 1H), 3.02 (tt, 1H), 3.18 (bs, 1H), 3.75 (bs, 1H), 4.22 (bs, 1H), 4.52 (s, 2H), 5.01 (bs, 2H), 6.38 (s, 1H), 7.09 (s, 1H), 7.59 (dd, 1H), 7.63 (d, 1H), 7.77 (dd, 1H), 8.01 (dd, 1H), 8.38 (d, 1H), 8.92 (dd, 1H)
MS (ESI): 548 ([M+H]⁺)

### 2-[5-Methyl-3-(trifluormethyl)-1H-pyrazol-1-yl]-1-[4-(4-{[(1-phenyl-1H-tetrazol-5-yl)sulfanyl]methyl}-1,3-thiazol-2-yl)piperidin-1-yl]ethanthion (I-15)

Zu einer Lösung von 2-[5-Methyl-3-(trifluormethyl)-1H-pyrazol-1-yl]-1-[4-(4-{[(1-phenyl-1H-tetrazol-5-yl)sulfanyl]methyl}-1,3-thiazol-2-yl)piperidin-1-yl]ethanon (433 mg) in Toluen (3 mL), wird bei Raumtemperatur 2,4-Bis(4-methoxyphenyl)-1,3,2,4-dithiadiphosphetan-2,4-disulfid (211 mg) gegeben. Die Reaktionsmischung wird 6 Stunden bei 60 °C gerührt. Nach Entfernen des Lösungsmittels unter vermindertem Druck wird der Rückstand chromatographisch gereinigt. Man erhält 2-[5-Methyl-3-(trifluormethyl)-1H-pyrazol-1-yl]-1-[4-(4-{[(1-phenyl-1H-tetrazol-5-yl)sulfanyl]methyl}-1,3-thiazol-2-yl)piperidin-1-yl]ethanthion (254 mg, 57 %).
logP (pH2.7): 3.82
1H NMR (CD3CN, 400 MHz): δₚₚₘ: 1.74 (m, 1H), 1.82 (m, 1H), 2.10-2.20 (m, 2H), 2.29 (s, 3H), 3.32 (td, 1H), 3.38 (m, 1H), 3.50 (m, 1H), 4.37 (m, 1H), 4.62 (s, 2H), 5.23 (s, 2H), 5.37 (m, 1H), 6.39 (s, 1H), 7.32 (s, 1H), 7.53-7.56 (m, 2H), 7.59-7.63 (m, 3H)

### MS (ESI): 565 ([M+H]+)

Die Messung der logP Werte erfolgte gemäß EEC Directive 79/831 Annex V.A8 durch HPLC (High Performance Liquid Chromatography) an reversed-phase Säulen (C 18), mit nachfolgenden Methoden: Die Bestimmung mit der LC-MS im sauren Bereich erfolgt bei pH 2,7 mit 0,1 % wässriger Ameisensäure und Acetonitril (enthält 0,1% Ameisensäure) als Eluenten, linearer Gradient von 10% Acetonitril bis 95% Acetonitril. Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).Die lambda-maX-Werte wurden an Hand der UV-Spektren von 200 nm bis 400 nm in den Maxima der chromatographischen Signale ermittelt.

### Verwendungsbeispiele

### Beispiel A: Phytophthora-Test (Tomate) / protektiv

| | |
|---|---|
| Lösungsmittel: | 49 Gewichtsteile N, N - Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von Phytophthora infestans inokuliert und stehen dann 24 h bei 100 rel. Feuchte und 22 °C. Anschließend werden die Pflanzen in einer Klimazelle bei ca. 96 % relativer Luftfeuchtigkeit und einer Temperatur von ca. 20 °C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen (I-1), (I-2), (I-3), (I-6), (I-7), (I-8), (I-9), (I-10) und (I-13) bei einer Konzentration an Wirkstoff von 500 ppm einen Wirkungsgrad von 70 % oder mehr.

### Beispiel B: Plasmopara-Test (Rebe) / protektiv

| | |
|---|---|
| Lösungsmittel : | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei ca. 20 °C und 100 % relativer Luftfeuchtigkeit. Anschließend werden die Pflanzen 4 Tage im Gewächshaus bei ca. 21 °C und ca. 90 % Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen (I-1), (I-4) und (I-9) bei einer Konzentration an Wirkstoff von 100 ppm einen Wirkungsgrad von 70 % oder mehr.

## Patentansprüche

1. Verbindungen der Formel (I), 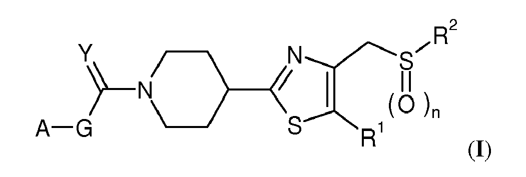 in welcher die Symbole folgende Bedeutungen haben:
A steht für Phenyl, das bis zu drei Substituenten enthalten kann, wobei die Substituenten unabhängig voneinander ausgewählt sind aus folgender Lis- te: Cyano, Nitro, Halogen, C₁-C₆-Alkyl, C₁-C₄-Halogenalkyl, C₃-C₆-Cycloalkyl, C₃-C₆- Halogencycloalkyl, C₄-C₁₀-Cyloalkylalkyl, C₄-C₁₀-Alkylcycloalkyl, C₅-C₁₀- Alkylcycloalkylalkyl, C₂-C₆-Alkenyl, C₂-C₆-Halogenalkenyl, C₂-C₆-Alkinyl, C₂-C₆- Halogenalkinyl, Tri(C₁-C₄-alkyl)silyl, Benzyl, Phenyl, Hydroxy, C₁-C₄-Alkoxy, C₁- C₄-Halogenalkoxy, OCH₂OCH₃, SH, C₁-C₄-Alkylthio, C₁-C₆-Halogenalkylthio, CHO, COOH, (C₁-C₄-Alkoxy)carbonyl, CONR³R⁴, CR³=NOR⁴, (C₁-C₄- Alkyl)carbonyl, (C₁-C₄-Halogenalkyl)carbonyl, (C₁-C₄-Alkyl)carbonyloxy, (C₁-C₄- Alkyl)carbonylthio, C₁-C₄-Alkylsulfinyl, C₁-C₄-Halogenalkylsulfinyl, C₁-C₄- Alkylsulfonyl, C₁-C₄-Halogenalkylsulfonyl, NR³R⁴, NR³COR⁴, SF₅, SO₂NR³R⁴, C₂- C₄-Alkoxyalkyl, oder 1-Methoxycyclopropyl, oder oder
A steht für einen heteroaromatischen Rest ausgewählt aus folgender Gruppe: Furan-2- yl, Furan-3-yl, Thiophen-2-yl, Thiophen-3-yl, Isoxazol-3-yl, Isoxazol-4-yl, Isoxazol- 5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol- 5-yl, Pyrazol-1-yl, Pyrazol-3-yl, Pyrazol-4-yl, Imidazol-1-yl, Imidazol-2-yl, Imida- zol-4-yl, Pyridin-2-yl, Pyridin-3-yl, Pyridin-4-yl, Pyridazin-3-yl, Pyridazin-4-yl, Py- rimidin-2-yl, Pyrimidin-4-yl oder Pyrimidin-5-yl, der bis zu drei Substituenten enthalten kann, wobei die Substituenten unabhängig voneinander ausgewählt sind aus folgender Liste: Substituenten am Kohlenstoff: Cyano, Nitro, Halogen, C₁-C₆-Alkyl, C₁-C₄-Halogenalkyl, C₃-C₆-Cycloalkyl, C₃-C₆- Halogencycloalkyl C₄-C₁₀-Cyloalkylalkyl, C₄-C₁₀-Alkylcycloalkyl, C₅-C₁₀- Alkylcycloalkylalkyl, C₂-C₆-Alkenyl, C₂-C₆-Halogenalkenyl, C₂-C₆-Alkinyl, C₂-C₆- Halogenalkinyl, Tri(C₁-C₄-alkyl)silyl, Benzyl, Phenyl, Hydroxy, C₁-C₄-Alkoxy, C₁- C₄-Halogenalkoxy, OCH₂OCH₃, SH, C₁-C₄-Alkylthio, C₁-C₆-Halogenalkylthio, CHO, COOH, (C₁-C₄-Alkoxy)carbonyl, CONR³R⁴, CR³=NOR⁴, (C₁-C₄- Alkyl)carbonyl, (C₁-C₄-Halogenalkyl)carbonyl, (C₁-C₄-Alkyl)carbonyloxy, (C₁-C₄- Alkyl)carbonylthio, C₁-C₄-Alkylsulfinyl, C₁-C₄-Halogenalkylsulfinyl, C₁-C₄-Alkyl- sulfonyl, C₁-C₄-Halogenalkylsulfonyl, NR³R⁴, NR³COR⁴, SF₅, SO₂NR³R⁴, C₂-C₄- Alkoxyalkyl oder 1-Methoxycyclopropyl, Substituenten am Stickstoff: Hydroxy, Cyano, NR³R⁴, C₁-C₆-Alkyl, C₁-C₄-Halogenalkyl, C₃-C₆-Cycloalkyl, C₃- C₆-Halogencycloalkyl, C₄-C₁₀-Alkylcyloalkyl, C₂-C₆-Alkenyl, C₂-C₆- Halogenalkenyl, C₂-C₆-Alkinyl oder C₂-C₆-Halogenalkinyl,
G steht für (C(R⁵)₂)ₚ, mit p = 1 oder 2, oder
G steht für NH, unter der Voraussetzung, dass G an ein Kohlenstoffatom von A gebun- den ist,
Y steht für Schwefel oder Sauerstoff,
R¹ steht für Wasserstoff, C₁-C₂-Alkyl, C₁-C₂-Halogenalkyl oder Halogen,
n = 0 bis 2,
R² steht für C₁-C₈-Alkyl, C₁-C₄-Halogenalkyl, C₂-C₆-Alkenyl, C₂-C₆-Alkynyl, Adaman- tan-1-yl oder Adamantan-2-yl, oder
R² steht für unsubstituiertes oder substituiertes C₃-C₁₀-Cycloalkyl, wobei die Substituenten unabhängig voneinander ausgewählt sind aus folgender Lis- te: Cyano, Halogen, C₁-C₆-Alkyl, C₁-C₆-Halogenalkyl, C₃-C₆-Cycloalkyl, C₂-C₆- Alkenyl, C₂-C₆-Halogenalkenyl, C₂-C₆-Alkinyl, C₂-C₆-Halogenalkinyl, Tri(C₁-C₄- alkyl)silyl, Phenyl, Hydroxy, Oxo, C₁-C₆-Alkoxy, C₁-C₆-Halogenalkoxy, C₂-C₆- Alkenyloxy, C₂-C₆-Alkinyloxy, C₁-C₆-Alkylthio oder C₁-C₆-Halogenalkylthio, oder
R² steht für unsubstituiertes oder substituiertes C₅-C₁₀-Cyloalkenyl, wobei die Substituenten unabhängig voneinander ausgewählt sind aus folgender Lis- te: Cyano, Halogen, C₁-C₆-Alkyl, C₁-C₆-Halogenalkyl, C₃-C₆-Cycloalkyl, C₂-C₆- Alkenyl, C₂-C₆-Halogenalkenyl, C₂-C₆-Alkinyl, C₂-C₆-Halogenalkinyl, Tri(C₁-C₄- alkyl)silyl, Phenyl, Hydroxy, Oxo, C₁-C₆-Alkoxy, C₁-C₆-Halogenalkoxy, C₂-C₆- Alkenyloxy, C₂-C₆-Alkinyloxy, C₁-C₆-Alkylthio oder C₁-C₆-Halogenalkylthio, oder
R² steht für unsubstituiertes oder substituiertes Phenyl, wobei die Substituenten unabhängig voneinander ausgewählt sind aus folgender Lis- te: Cyano, Nitro, Halogen, C₁-C₆-Alkyl, C₁-C₄-Halogenalkyl, C₃-C₆-Cycloalkyl, C₃-C₆- Halogencycloalkyl, C₄-C₁₀-Cycloalkylalkyl, C₄-C₁₀-Halogencycloalkylalkyl, C₄-C₁₀- Alkylcycloalkyl, C₅-C₁₀-Alkylcycloalkylalkyl, C₄-C₁₀-Cycloalkoxyalkyl, C₂-C₆- Alkenyl, C₂-C₆-Halogenalkenyl, C₃-C₈-Cycloalkenyl, C₃-C₈-Halocycloalkenyl, C₂- C₆-Alkinyl, C₂-C₆-Halogenalkinyl, C₂-C₆-Alkoxyalkyl, C₂-C₆-Halogenalkoxyalkyl, C₃-C₈-Alkoxyalkoxyalkyl, Tri(C₁-C₄-alkyl)silyl, Benzyl, Phenyl, Hydroxy, C₁-C₆- Alkoxy, C₂-C₆-Alkoxyalkoxy, C₁-C₆-Halogenalkoxy, C₂-C₆-Alkenyloxy, C₂-C₆- Halogenalkenyloxy, C₂-C₆-Alkinyloxy, C₂-C₆-Halogenalkinyloxy, C₃-C₆- Cycloalkoxy, C₃-C₆-Cycloalkyloxy, C₃-C₆-Halocycloalkoxy, C₄-C₁₀- Cycloalkylalkyloxy, NR³R⁴, SH, SF₅, C₁-C₆-Alkylthio, C₁-C₆-Halogenalkylthio, C₃- C₆-Cycloalkylthio, CHO, COOH, (C₁-C₆-Alkoxy)carbonyl, CONR³R⁴, CR³=NOR⁴, (C₁-C₆-Alkyl)carbonyl, (C₁-C₆-Halogenalkyl)carbonyl, (C₁-C₆-Alkyl)carbonyloxy, (C₁-C₆-Halogenalkyl)carbonyloxy, (C₁-C₆-Alkyl)carbonylthio, C₁-C₆-Alkylsulfinyl, C₁-C₆-Halogenalkylsulfinyl, C₁-C₆-Alkylsulfonyl, C₁-C₆-Halogenalkylsulfonyl, NR³COR⁴ oder SO₂NR³R⁴, oder
R² steht für gesättigtes oder teilweise oder vollständig ungesättigtes, unsubstituiertes oder substituiertes, Naphthyl oder Indenyl, wobei die Substituenten unabhängig voneinander ausgewählt sind aus folgender Lis- te: Cyano, Nitro, Halogen, C₁-C₆-Alkyl, C₁-C₄-Halogenalkyl, C₃-C₆-Cycloalkyl, C₂-C₆- Alkenyl, C₂-C₆-Halogenalkenyl, C₂-C₆-Alkinyl, C₂-C₆-Halogenalkinyl, Tri(C₁-C₄- alkyl)silyl, Benzyl, Phenyl, Hydroxy, C₁-C₆-Alkoxy, C₁-C₆-Halogenalkoxy, C₂-C₆- Alkenyloxy, C₂-C₆-Alkinyloxy, C₁-C₆-Alkylthio oder C₁-C₆-Halogenalkylthio, oder
R² steht für einen unsubstituierten oder substituierten 5- oder 6-gliedrigen Heteroaryl- rest, wobei die Substituenten unabhängig voneinander ausgewählt sind aus folgender Liste: Substituenten am Kohlenstoff: Cyano, Nitro, Halogen, C₁-C₆-Alkyl, C₁-C₃- Halogenalkyl, C₃-C₆-Cycloalkyl, C₃-C₆-Halogencycloalkyl, C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆- Halogenalkenyl, C₂-C₆-Alkinyl, C₂-C₆-Halogenalkinyl, Tri(C₁-C₄-alkyl)silyl, Ben- zyl, Phenyl, Hydroxy, C₁-C₄-Alkoxy, C₁-C₄-Halogenalkoxy, OCH₂OCH₃, SH, C₁- C₄-Alkylthio, C₁-C₆-Halogenalkylthio, COOH, (C₁-C₄-Alkoxy)carbonyl, CONR³R⁴, (C₁-C₄-Alkyl)carbonyl, (C₁-C₄-Halogenalkyl)carbonyl, (C₁-C₄-Alkyl)carbonyloxy, (C₁-C₄-Alkyl)carbonylthio, C₁-C₄-Alkylsulfinyl, C₁-C₄-Halogenalkylsulfinyl, C₁-C₄- Alkylsulfonyl, C₁-C₄-Halogenalkylsulfonyl, NR³R⁴, NR³COR⁴, SF₅, SO₂NR³R⁴, C₂- C₄-Alkoxyalkyl oder 1-Methoxycyclopropyl, Substituenten am Stickstoff: Cyano, C₁-C₆-Alkyl, C₁-C₃-Halogenalkyl, C₃-C₆- Cycloalkyl, C₃-C₆-Halogencycloalkyl, C₄-C₁₀-Alkylcyloalkyl, C₂-C₆-Alkenyl, C₂- C₆-Halogenalkenyl, C₂-C₆-Alkinyl, C₂-C₆-Halogenalkinyl oder Phenyl, oder
R² steht für benzokondensiertes unsubstituiertes oder substituiertes 5- oder 6- gliedriges Heteroaryl, wobei die Substituenten unabhängig voneinander ausgewählt sind aus folgender Liste: Substituenten am Kohlenstoff: Cyano, Nitro, Halogen, C₁-C₆-Alkyl, C₁-C₃- Halogenalkyl, C₃-C₆-Cycloalkyl, C₃-C₆-Halogencycloalkyl C₄-C₁₀-Cycloalkylalkyl, C₄-C₁₀-Alkylcyloalkyl, C₅-C₁₀-Alkylcycloalkylalkyl, C₂-C₆-Alkenyl, C₂-C₆- Halogenalkenyl, C₂-C₆-Alkinyl, C₂-C₆-Halogenalkinyl, Tri(C₁-C₄-alkyl)silyl, Ben- zyl, Phenyl, Hydroxy, C₁-C₄-Alkoxy, C₁-C₄-Halogenalkoxy, OCH₂OCH₃, SH, C₁- C₄-Alkylthio, C₁-C₆-Halogenalkylthio, COOH, (C₁-C₄-Alkoxy)carbonyl, CONR³R⁴, (C₁-C₄-Alkyl)carbonyl, (C₁-C₄-Halogenalkyl)carbonyl, (C₁-C₄-Alkyl)carbonyloxy, (C₁-C₄-Alkyl)carbonylthio, C₁-C₄-Alkylsulfinyl, C₁-C₄-Halogenalkylsulfinyl, C₁-C₄- Alkylsulfonyl, C₁-C₄-Halogenalkylsulfonyl, NR³R⁴, NR³COR⁴, SF₅, SO₂NR³R⁴, C₂- C₄-Alkoxyalkyl oder 1-Methoxycyclopropyl, Substituenten am Stickstoff: Cyano, C₁-C₆-Alkyl, C₁-C₃-Halogenalkyl, C₃-C₆- Cycloalkyl, C₃-C₆-Halogencycloalkyl, C₄-C₁₀-Alkylcyloalkyl, C₂-C₆-Alkenyl, C₂- C₆-Halogenalkenyl, C₂-C₆-Alkinyl, C₂-C₆-Halogenalkinyl oder Phenyl, oder
R² steht für unsubstituiertes oder substituiertes C₅-C₁₅-Heterocyclyl, wobei mögliche Substituenten unabhängig voneinander ausgewählt sind aus folgen- der Liste: Substituenten am Kohlenstoff: Cyano, Nitro, Halogen, C₁-C₆-Alkyl, C₁-C₃- Halogenalkyl, C₃-C₆-Cycloalkyl, C₃-C₆-Halogencycloalkyl C₄-C₁₀-Cycloalkylalkyl, C₄-C₁₀-Alkylcyloalkyl, C₅-C₁₀-Alkylcycloalkylalkyl, C₂-C₆-Alkenyl, C₂-C₆- Halogenalkenyl, C₂-C₆-Alkinyl, C₂-C₆-Halogenalkinyl, Tri(C₁-C₄-alkyl)silyl, Ben- zyl, Phenyl, Hydroxy, C₁-C₄-Alkoxy, C₁-C₄-Halogenalkoxy, OCH₂OCH₃, SH, C₁- C₄-Alkylthio, C₁-C₆-Halogenalkylthio, COOH, (C₁-C₄-Alkoxy)carbonyl, CONR³R⁴, (C₁-C₄-Alkyl)carbonyl, (C₁-C₄-Halogenalkyl)carbonyl, (C₁-C₄-Alkyl)carbonyloxy, (C₁-C₄-Alkyl)carbonylthio, C₁-C₄-Alkylsulfinyl, C₁-C₄-Halogenalkylsulfinyl, C₁-C₄- Alkylsulfonyl, C₁-C₄-Halogenalkylsulfonyl, NR³R⁴, NR³COR⁴, SF₅, SO₂NR³R⁴, C₂- C₄-Alkoxyalkyl oder 1-Methoxycyclopropyl, Substituenten am Stickstoff: Cyano, C₁-C₆-Alkyl, C₁-C₃-Halogenalkyl, C₃-C₆- Cycloalkyl, C₃-C₆-Halogencycloalkyl, C₄-C₁₀-Alkylcyloalkyl, C₂-C₆-Alkenyl, C₂- C₆-Halogenalkenyl, C₂-C₆-Alkinyl, C₂-C₆-Halogenalkinyl oder Phenyl,
R³, R⁴ stehen unabhängig voneinander für Wasserstoff, C₁-C₄-Alkyl, C₁-C₃-Halogenalkyl, C₃-C₆-Cycloalkyl, Benzyl oder Phenyl,
R⁵ ist gleich oder verschieden unabhängig voneinander Wasserstoff, C₁-C₂-Alkyl oder C₁-C₂-Halogenalkyl,
sowie agrochemisch wirksame Salze davon.

2. Verbindungen der Formel (**I**) nach Anspruch 1, in welcher die Symbole folgende Bedeutungen haben,
A steht für Phenyl, das bis zu zwei Substituenten enthalten kann, wobei die Substituenten unabhängig voneinander ausgewählt sind aus folgender Lis- te: Cyano, Halogen, Methyl, Ethyl, Propyl, 1-Methylethyl, 1,1-Dimethylethyl, CF₃, CHF₂, C₂F₅, CCl₃, OMe, OCF₃, OCHF₂ oder OC₂F₅, oder
A steht für einen heteroaromatischen Rest ausgewählt aus folgender Gruppe: Pyrazol- 1-yl, Pyrazol-3-yl, Pyrazol-4-yl, Pyridin-2-yl, Pyridin-3-yl oder Pyridin-4-yl, der bis zu zwei Substituenten enthalten kann, wobei die Substituenten unabhängig voneinander ausgewählt sind aus folgender Liste: Substituenten am Kohlenstoff: Cyano, Halogen, Methyl, Ethyl, Propyl, 1- Methylethyl, 1,1-Dimethylethyl, CF₃, CHF₂, C₂F₅, CCl₃, OMe, OCF₃, OCHF₂ oder OC₂F₅, Substituenten am Stickstoff: Methyl, Ethyl oder CF₃,
G steht für CH₂,
Y steht für Schwefel oder Sauerstoff,
R¹ steht für Wasserstoff,
n = 0 bis 2,
R² steht für Methyl, Ethyl, 1-Methylethyl, 1,1-Dimethylethyl, Propyl, Butyl, Pentyl, Hexyl, Adamantan-1-yl oder Adamantan-2-yl, oder
R² steht für C₅-C₈-Cycloalkyl, das bis zu vier Substituenten enthalten kann, wobei die Substituenten unabhängig voneinander ausgewählt sind aus folgender Lis- te: Methyl, Ethyl, 1-Methylethyl oder 1,1-Dimethylethyl, oder
R² steht für C₅-C₈-Cycloalkenyl, das bis zu vier Substituenten enthalten kann, wobei die Substituenten unabhängig voneinander ausgewählt sind aus folgender Lis- te: Methyl, Ethyl, 1-Methylethyl oder 1,1-Dimethylethyl, oder
R² steht für Phenyl, das bis zu zwei Substituenten enthalten kann, wobei die Substituenten unabhängig voneinander ausgewählt sind aus folgender Lis- te: Cyano, Nitro, Halogen, Methyl, Ethyl, 1-Methylethyl, 1,1-Dimethylethyl, CF₃, CHF₂, C₂F₅, CCl₃, -CH=CH₂, -CH₂CH=CH₂, Benzyl, Phenyl, OMe, OCF₃, OCHF₂, C₂F₅, SMe oder SCF₃, oder
R² steht für Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8- Tetrahydronaphthalen-2-yl, Decalin-1-yl, Decalin-2-yl, 1H-Inden-1-yl, 1H-Inden-2- yl, 1H-Inden-3-yl, 1H-Inden-4-yl, 1H-Inden-5-yl, 1H-Inden-6-yl, 1H-Inden-7-yl, In- dan-1-yl, Indan-2-yl, Indan-3-yl, Indan-4-yl oder Indan-5-yl, das bis zu zwei Substituenten enthalten kann, wobei die Substituenten unabhängig voneinander ausgewählt sind aus folgender Liste: Cyano, Nitro, Halogen, Methyl, Ethyl, 1-Methylethyl, 1,1-Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃, oder
R² steht für Furan-2-yl, Furan-3-yl, Thiophen-2-yl, Thiophen-3-yl, Isoxazol-3-yl, Isoxa- zol-4-yl, Isoxazol-5-yl, Pyrrol-1-yl, Pyrrol-2-yl, Pyrrol-3-yl, Oxazol-2-yl, Oxazol-4- yl, Oxazol-5-yl, Thiazol-2-yl, Thiazol-4-yl, Thiazol-5-yl, Isothiazol-3-yl, Isothiazol- 4-yl, Isothiazol-5-yl, Pyrazol-1-yl, Pyrazol-3-yl, Pyrazol-4-yl, Imidazol-1-yl, Imida- zol-2-yl, Imidazol-4-yl, 1,2,4-Oxadiazol-3-yl, 1,2,4-Oxadiazol-5-yl, 1,3,4- Oxadiazol-2-yl, Tetrazol-5-yl, 1,2,4-Thiadiazol-3-yl, 1,2,4-Thiadiazol-5-yl, 1,3,4- Thiadiazol-2-yl, 1,2,3-Triazol-1-yl, 1,2,3-Triazol-2-yl, 1,2,3-Triazol-4-yl, 1,2,4- Triazol-1-yl, 1,2,4-Triazol-3-yl, 1,2,4-Triazol-4-yl, Pyridin-2-yl, Pyridin-3-yl, Pyri- din-4-yl, Pyridazin-3-yl, Pyridazin-4-yl, Pyrimidin-2-yl, Pyrimidin-4-yl, Pyrimidin- 5-yl, Pyrazin-2-yl, 1,3,5-Triazin-2-yl oder 1,2,4-Triazin-3-yl, das bis zu zwei Substituenten enthalten kann, wobei die Substituenten unabhängig voneinander ausgewählt sind aus folgender Liste: Substituenten am Kohlenstoff: Cyano, Nitro, Halogen, Methyl, Ethyl, 1-Methylethyl, 1,1-Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃, Substituenten am Stickstoff: Methyl, Ethyl, 1-Methylethyl, 1,1-Dimethylethyl oder Phenyl, oder
R² steht für Indol-1-yl, Indol-2-yl, Indol-3-yl, Indol-4-yl, Indol-5-yl, Indol-6-yl, Indol-7- yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, In- dazol-2-yl, 1-Benzofuran-2-yl, 1-Benzofuran-3-yl, 1-Benzofuran-4-yl, 1- Benzofuran-5-yl, 1-Benzofuran-6-yl, 1-Benzofuran-7-yl, 1-Benzothiophen-2-yl, 1- Benzothiophen-3-yl, 1-Benzothiophen-4-yl, 1-Benzothiophen-5-yl, 1- Benzothiophen-6-yl, 1-Benzothiophen-7-yl, 1,3-Benzothiazol-2-yl, 1,3- Benzothiazol-4-yl, 1,3-Benzothiazol-5-yl, 1,3-Benzothiazol-6-yl, 1,3-Benzothiazol- 7-yl, 1,3-Benzoxazol-2-yl, 1,3-Benzoxazol-4-yl, 1,3-Benzoxazol-5-yl, 1,3- Benzoxazol-6-yl, 1,3-Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochi- nolin-3-yl, Isochinolin-4-yl, Isochinolin-5-yl, Isochinolin-6-yl, Isochinolin-7-yl oder Isochinolin-8-yl, das bis zu zwei Substituenten enthalten kann, wobei die Substituenten unabhängig voneinander ausgewählt sind aus folgender Liste: Substituenten am Kohlenstoff: Cyano, Nitro, Halogen, Methyl, Ethyl, 1-Methylethyl, 1,1-Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃, Substituenten am Stickstoff: Methyl, Ethyl, 1-Methylethyl, 1,1-Dimethylethyl oder Phenyl, oder
R² steht für Piperidin-1-yl, Piperidin-2-yl, Piperidin-3-yl, Piperidin-4-yl, Piperazin-1-yl, Piperazin-2-yl, Piperazin-3-yl, Morpholin-1-yl, Morpholin-2-yl, Morpholin-3-yl, Tetrahydropyran-2-yl, Tetrahydropyran-3-yl, Tetrahydropyran-4-yl, 1,2,3,4- Tetrahydrochinolin-1-yl, 1,2,3,4-tetrahydroisochinolin-2-yl, 1,2,3,4- Tetrahydrochinoxalin-1-yl, Indolin-1-yl, Isoindolin-2-yl, Decahydrochinolin-1-yl oder Decahydroisochinolin-2-yl, das bis zu zwei Substituenten enthalten kann, wobei mögliche Substituenten unabhängig voneinander ausgewählt sind aus folgen- der Liste: Substituenten am Kohlenstoff: Cyano, Nitro, Halogen, Methyl, Ethyl, 1-Methylethyl, 1,1-Dimethylethyl, CF₃, CHF₂, C₂F₅, CCl₃, -CH=CH₂, -CH₂CH=CH₂, Tri(methyl)silyl, Benzyl, Phenyl, OMe, OEt, O*iso*Pr, OCF₃, OCHF₂, OC₂F₅, SMe oder SCF₃,
Substituenten am Stickstoff: Methyl, Ethyl, 1-Methylethyl, 1,1-Dimethylethyl oder Phenyl,
sowie agrochemisch wirksame Salze davon.

3. Verbindungen der Formel **(I)** nach einem oder mehreren der Ansprüche 1 bis 2,
in welcher die Symbole folgende Bedeutungen haben,
A steht für 5-Methyl-3-(trifluormethyl)-1H-pyrazol-1-yl,
G steht für CH₂,
Y steht für Schwefel oder Sauerstoff,
R¹ steht für Wasserstoff,
n = 0 bis 2,
R² steht für Naphthalen-1-yl, Adamantan-1-yl, Cyclohexyl, 1-Phenyl-tetrazol-5-yl oder Chinolin-8-yl,
sowie agrochemisch wirksame Salze davon.

4. Verfahren zur Bekämpfung von pflanzenpathogenen Schadpilzen, **dadurch gekennzeichnet, dass** man Verbindungen der Formel **(I)** gemäß einem oder mehreren der Ansprüche 1 bis 3, auf die pflanzenpathogenen Schadpilze und/oder deren Lebensraum ausbringt.

5. Mittel zur Bekämpfung von pflanzenpathogenen Schadpilzen, **gekennzeichnet durch** einen Gehalt an mindestens einer Verbindung der Formel **(I)** gemäß einem oder mehreren der Ansprüche 1 bis 3, neben Streckmitteln und/oder oberflächenaktiven Stoffen.

6. Verwendung von Thiazolylpiperidin Derivaten der Formel **(I),** gemäß einem oder mehreren der Ansprüche 1 bis 3 zur Bekämpfung von pflanzenpathogenen Schadpilzen.

7. Verfahren zur Herstellung von Mitteln zur Bekämpfung von pflanzenpathogenen Schadpilzen, **dadurch gekennzeichnet, dass** man Thiazolylpiperidin Derivate der Formel **(I)** gemäß einem oder mehreren der Ansprüche 1 bis 3 mit Streckmitteln und/oder oberflächenaktiven Stoffen vermischt.

8. Verfahren zum Herstellen der Verbindungen der Formel **(I)** umfassend wenigstens einen der
folgenden Schritte (a) bis (g): 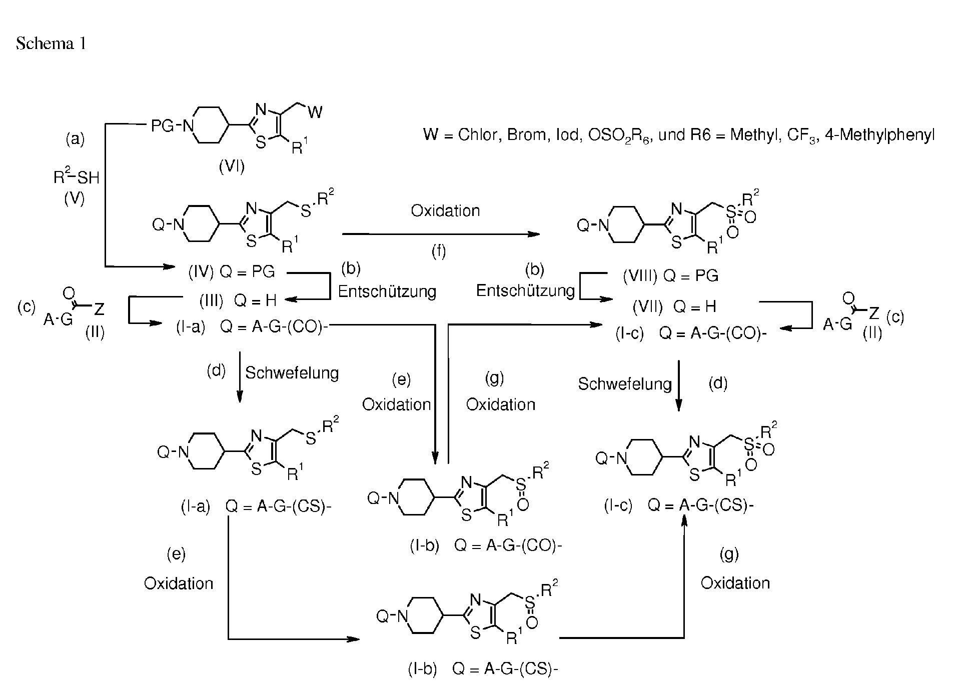 worin R¹, R², A und G die in Anspruch 1 angegebenen Bedeutungen haben.

9. Verbindungen der Formel **(VI-a)** 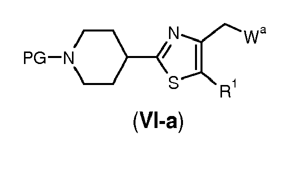 in denen
PG für Acetyl, C₁-C₄-Alkoxycarbonyl, Benzyl oder Benzyloxycarbonyl steht, W^{a} für Brom oder Iod steht,
R¹ die in Anspruch 1 angegebenen Bedeutungen hat,
sowie Salze davon.

10. Verbindungen der Formel **(IV)** 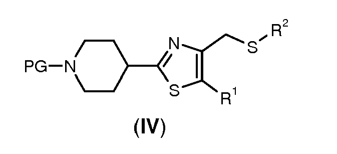 in denen
PG für Acetyl, C₁-C₄-Alkoxycarbonyl, Benzyl oder Benzyloxycarbonyl steht,
R¹ und R² die in Anspruch 1 angegebenen Bedeutungen haben,
sowie Salze davon,
ausgenommen die Verbindung tert-Butyl-4-[4-({[4-(methylsulfonyl)phenyl]sulfanyl}methyl)-1,3-thiazol-2-yl]piperidin-1-carboxylat.

11. Verbindungen der Formel **(III)** 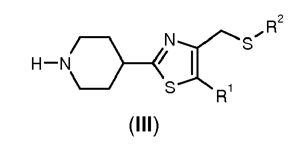 in denen
R¹ und R² die in Anspruch 1 angegebenen Bedeutungen haben,
sowie Salze davon.

12. Verwendung von Verbindungen der Formel **(I)** gemäß den Ansprüchen 1 bis 3 zur Behandlung von Saatgut.

13. Verwendung von Verbindungen der Formel **(I)** gemäß den Ansprüchen 1 bis 3 zur Behandlung von transgenen Pflanzen.

14. Verwendung von Verbindungen der Formel **(I)** gemäß den Ansprüchen 1 bis 3 zur Behandlung von transgenem Saatgut.
